(19) Europäisches Patentamt
European Patent Office
Office européen des brevets

(11) **EP 3 162 372 B1**

(12) **EUROPEAN PATENT SPECIFICATION**

(45) Date of publication and mention
of the grant of the patent:
**17.02.2021 Bulletin 2021/07**

(51) Int Cl.:
*A61K 35/12* (2015.01)      *A61K 39/395* (2006.01)
*A61P 9/10* (2006.01)        *A61P 9/00* (2006.01)

(21) Application number: **15812792.8**

(22) Date of filing: **26.06.2015**

(86) International application number:
**PCT/KR2015/006588**

(87) International publication number:
**WO 2015/199499 (30.12.2015 Gazette 2015/52)**

(54) **COMPOSITION FOR USE IN THE TREATMENT OF ISCHEMIC DISEASES CONTAINING NEURAL PROGENITOR CELLS AS ACTIVE INGREDIENT**

ZUSAMMENSETZUNG ZUR BEHANDLUNG VON ISCHÄMISCHEN KRANKHEITEN ODER NEUROGENER ENTZÜNDUNG, MIT NEURALEN VORLÄUFERZELLEN ODER SEKRETOM DAVON ALS WIRKSTOFF

COMPOSITION POUR LE TRAITEMENT DE MALADIES ISCHÉMIQUES OU DE L'INFLAMMATION NEUROGÈNE, CONTENANT, COMME AGENT ACTIF, DES CELLULES PROGÉNITRICES NEURALES OU LEUR SÉCRÉTOME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2014 KR 20140080009**

(43) Date of publication of application:
**03.05.2017 Bulletin 2017/18**

(60) Divisional application:
**18176352.5 / 3 403 658**

(73) Proprietor: **S-BIOMEDICS**
**Seoul 04797 (KR)**

(72) Inventors:
• **KIM, Dong Wook**
  **Seoul 121-766 (KR)**
• **KIM, Han Soo**
  **Goyang-si**
  **Gyeonggi-do 411-370 (KR)**

(74) Representative: **Plougmann Vingtoft a/s**
**Strandvejen 70**
**2900 Hellerup (DK)**

(56) References cited:
**WO-A1-2008/070513    KR-A- 20140 071 512**

• **MACAS JADRANKA ET AL: "Increased generation of neuronal progenitors after ischemic injury in the aged adult human forebrain", JOURNAL OF NEUROSCIENCE, SOCIETY FOR NEUROSCIENCE, US, vol. 26, no. 50, 30 November 2006 (2006-11-30), pages 13114-13119, XP009502581, ISSN: 0270-6474, DOI: 10.1523/JNEUROSCI.4667-06.2006**
• **FOX GERARD B ET AL: "The modulations of NCAM polysialylation state that follow transient global ischemia are brief on neurons but enduring on glia", JOURNAL OF NEUROPATHOLOGY AND EXPERIMENTAL NEUROLOGY, LIPPINCOTT WILLIAMS AND WILKINS, NEW YORK, NY, vol. 60, no. 2, 31 January 2001 (2001-01-31), pages 132-140, XP009502583, ISSN: 0022-3069, DOI: 10.1093/JNEN/60.2.132**
• **IWAI MASANORI ET AL: "Three steps of neural stem cells development in gerbil dentate gyrus after transient ischemia", JOURNAL OF CEREBRAL BLOOD FLOW & METABOLISM, NATURE PUBLISHING GROUP, US, vol. 22, no. 4, 31 March 2002 (2002-03-31) , pages 411-419, XP009502582, ISSN: 0271-678X, DOI: 10.1097/00004647-200204000-00005**
• **DATABASE WPI Week 201410 Thomson Scientific, London, GB; AN 2012-R77360 XP002777022, & KR 2012 0136638 A (UNIV YONSEI IND ACADEMIC COOP FOUND) 20 December 2012 (2012-12-20)**

- **DIBAJNIA ET AL.: 'Role of neural precursor cells in promoting repair following stroke' ACTA PHARMACOLOGICA SINICA vol. 34, no. 1, 2013, ISSN 1671-4083 pages 78 - 90, XP055248735**
- **INDUSTRY-ACADEMIC COOPERATION FOUNDATION: 'Establishment of Clinically Applicable X Linked Adrenoleukodystrophy Patient Specific Reprogrammed Stem Cells' HEALTH & MEDICAL TECHNOLOGY R&D PROGRAM (2012) REPORT XP055397914**
- **KIM, DAE - SUNG ET AL.: 'Highly Pure and Expandable PSA-NCAM-Positive Neural' PLOS ONE vol. 7, no. 7, July 2012, page E39715, XP055248737**

Remarks:

The file contains technical information submitted after the application was filed and not included in this specification

**Description**

Technical Field

The present invention relates to a composition for use in the treatment of ischemic disease containing polysialylated neural cell adhesion molecule (PSA-NCAM)-positive neural precursor cells as active ingredient.

Background Art

Stem cells are regarded as a promising therapeutic candidate material for various diseases due to the multipotency thereof. For example, mesenchymal stem cells (MSCs) release many nutritional factors that can be easily obtained and isolated and achieve the promotion of angiogenesis and the inhibition of inflammation (Caplan, A. I., & Dennis, J. E. (2006). Journal of Cellular Biochemistry, 98, 1076-1084). These characteristics of MSCs have been considered in studies for the application to the treatment of a number of human diseases. Recent studies have found that MSCs contribute to the tissue repair in a large number of animal models and human clinical treatments (Chen, J., Li, Y., Katakowski, M., et al. (2003). Journal of Neuroscience Research, 73, 778-786; Kopen, G. C., Prockop, D. J., & Phinney, D. G. (1999). Proceedings of the National Academy of Sciences, 96, 10711-10716). Several reports stated the *in vitro* differentiation ability of MSC into the neural lineage (Bae, K. S., Park, J. B., Kim, H. S., Kim, D. S., Park, D. J., & Kang, S. J. (2011). Yonsei Medical Journal, 52, 401-412) and astrocytes (Kopen, G. C., Prockop, D. J., & Phinney, D. G. (1999). Proceedings of the National Academy of Sciences, 96, 10711-10716), but there is no definite evidence as to what functions the differentiated cells perform *in vivo.* It seems that the favorable effect of MSCs is induced by paracrine mechanisms rather than cell replacement, and therefore, the transplantation of MSCs would have temporary and limited effects but not the alleviation maintained for a long period of time (Cho, S. R., Kim, Y. R., Kang, H. S., et al. (2009). Cell Transplantion, 18, 1359-1368).

In contrast, embryonic stem cells (ESCs) may differentiate into all particular cell types derived from three embryonic germ layers, and have a strong self-renewal ability. Noticeably, neural precursor cells (NPCs) derived from ESCs, first, differentiate into a particular cell type of neural lineage cells including neural cells, astrocytes, and oligodendrocyte, and thus are considered to be a cell source for repair of brain tissues. These cells secrete some factors for promoting the survival and proliferation of endogenous neural precursor cells (Capone, C., Frigerio, S., Fumagalli, S., et al. (2007). PLoSOne, 7, e373). However, it has not yet been known how NPCs differentiated from ESCs or the culture liquid of NPCs contribute to the improvement of functions after transplantation in disease models.

KIM, DAE - SUNG ET AL.,"Highly Pure and Expandable PSA-NCAM-Positive Neural",PLOS ONE, vol. 7, no. 7, July 2012, discloses PSA-NCAM-positive neural precursor cells and to their beneficial effects in cell therapy for neurodegenerative disorders.

DIBAJNIA ET AL., "Role of neural precursor cells in promoting repair following stroke", ACTA PHARMACOLOGICA SINICA, vol. 34, no. 1, 2013, pages 78-90, discloses the role of neural precursor cells in promoting repair following stroke, e.g. ischemic stroke.

MACAS JADRANKA ET AL., "Increased generation of neuronal progenitors after ischemic injury in the aged adult human forebrain", JOURNAL OF NEUROSCIENCE, SOCIETY FOR NEUROSCIENCE, US, vol. 26, no. 50, 30 November 2006, pages 13114-13119, discloses e.g. an increased generation of PSA-NCAM-positive neural precursor cells after ischemic injury in the aged adult human forebrain.

FOX GERARD B ET AL: "The modulations of NCAM polysialylation state that follow transient global ischemia are brief on neurons but enduring on glia", JOURNAL OF NEUROPATHOLOGY AND EXPERIMENTAL NEUROLOGY, LIPPINCOT WILLIAMS AND WILKINS, NEW YORK, vol. 60, no. 2, 31 January 2001,pages 132-140, discloses an adaptive process following an ishemic event consisting of polysialylated NCAM expression in the ischemic gerbil hippocampus as a model for neurotrophic-dependent repair/remodeling ensuing the following transient interruption of blood flow.

IWAI MASANORI ET AL., "Three steps of neural stem cells development in gerbil dentate gyrus after transient ischemia", JOURNAL OF CEREBRAL BLOOD FLOW & METABOLISM, NATURE PUBLISHING GROUP, US, vol. 22, no. 4, 31 March 2002, pages 411-419, discloses e.g. three steps of neural stem cell development in gerbil dentate gyrus after transient ischemia, reporting the increased development and migration of PSA-NCAM-positive cells in the subgranular zone after ischemia as sign of peristent neurogenesis following transient global ischemia.

DATABASE WPI, Week 201410, Thomson Scientific, London, GB; AN 2012-R77360 & KR 2012 0136638, 20 December 2012, discloses e.g. a method for separating neural precursor cells from pluripotent stem cells by obtaining cell population, contacting the cell population with polysialated neural cell adhesion molecule (PSA-NCAM) antibody, separating the cell united with the antibody and differentiating the separated cells into neural rosette.

WO 2008/070513 A1 discloses e.g. the modulation of PSA-NCAM as a regulator in the treatment of ocular disease.

Detailed Description of the Invention

Technical Problem

The present inventors endeavored to develop a fundamental method for treating ischemic disease or neuroinflammatory disease. As a result, the present inventors found, when neural progenitor cells expressing PSA-NCAM, which is a nerve adhesibe molecule on the cell surface, are injected into the lesion site, neuroregeneration is enhanced, vascularization is promoted and the inflammatory reaction is suppressed, so that ischemic diseases caused by vascular damage and nerve tissue damage from inflammation are treated efficiently, thereby completing the present invention. In addition, as an approach different from stem cell transplantation, it is possible to reduce the ischemic injury area and restore the nerve function by administering to the lesion site of the neuroepithelial cell secretory proteins (secretome), thereby regaining the lost function in neuroinflammatory or neurodegenerative diseases such as ischemic diseases and inflammation, Thereby confirming that the disease can be effectively treated, thereby completing the present invention.

Accordingly, an aspect of the present invention is to provide a composition for use in the treatment of ischemic disease, the composition containing polysialylated neural cell adhesion molecule (PSA-NCAM)-positive neural precursor cells as an active ingredient, wherein the PSA-NCAM-positive neural precursor cells are separate from neural rosettes differentiated from pluripotent stem cells, wherein the pluripotent stem cells are embryonic stem cells or induced pluripotent stem cells (iPSCs), wherein the ischemic disease is selected from the group consisting of ischemic cerebrovascular disease, ischemic heart disease, myocardial infarction, angina pectoris, lower limb artery ischemic disease, and distal limb ischemic disease.

Other purposes and advantages of the present invention will become more obvious with the following detailed description of the invention, claims, and drawings.

Technical Solution

In accordance with an aspect of the present invention, there is provided a composition for use in the treatment of ischemic disease, the composition containing poly-sialylated neural cell adhesion molecule (PSA-NCAM)-positive neural precursor cells as an active ingredient.

According to the present invention, the composition for use according to the present invention restores blood vessels of ischemic tissues or inhibits inflammatory responses in a subject with ischemic disease, and thus, suppresses the development of symptoms of the diseases, or removes or relieves the diseases. Therefore, the composition of the present invention *per se* may be a composition for use in the treatment of ischemic disease. As used herein, the term "treatment" or "treatment agent" includes a meaning of "treatment aid" or "treatment adjuvant".

According to the present invention, the composition for use according to the present invention significantly increases an angiogenesis-inducing factor, angiopoietin-1, and greatly increases the number of blood vessels and the concentration in microvessels at sites of administration (or transplantation). Therefore, the composition for use according to the present invention can be used as a composition for efficiently treating ischemic disease, wherein the composition restores blood vessel tissues or increases the number of blood vessels in a subject having a reduced blood flow rate caused by the loss of blood vessel tissues, deficiency of angiogenesis, or formation of abnormal blood vessels, thereby restoring the blood flow rate.

According to the present invention, the composition for use according to the present invention inhibits the activation of reactive microglial cells and astrocytes in administered (or transplanted) neural tissues. Microglial cells perform a primary immune function in the central nervous system, and the activated microglial cells, unlike microglial cells in a normal state, perform active phagocytosis and cell proliferation, and generate inflammation-mediated materials through the expression of genes, such as cytokines (e.g., TNF-$\alpha$, IL-1$\beta$, and IL-6), chemokines, inducible nitric oxide synthase (iNOS), and cyclooxygenase-2 (COX-2). Activated astrocytes also secrete inflammatory cytokines, such as IL-6, TGF-$\beta$, LIF, or IL-1, and the secreted cytokines again activate microglial cells and astrocytes, thereby aggravating environments in tissues. Therefore, the composition for use according to the present invention, which inhibits the activation of microglial cells and astrocytes, can effectively block the damage of neural tissues due to inflammatory responses.

As disclosed herein, the composition for use according to the present invention increases the expression of angiopoietin-1.

As used herein, the term "increasing expression" refers to significantly increasing the expression of a gene or a protein so as to be measurable, compared with normal persons, and more specifically, the term refers to being an expression level of 130% or more compared with controls.

As disclosed herein, the composition for use according to the present invention inhibits the activation of glial cells or astrocytes.

As used herein, the term "inhibiting activation" refers to an *in vivo* transformation that causes deteriorations in the number, functions, and activation of glial cells or astrocytes, and for example, refers to significantly decreasing the expression of a specific marker (e.g., CD68 or GFAP) of those cells, being impossible to detect the expression, or making the expression an insignificant level.

According to an embodiment of the present invention, the PSA-NCAM-positive neural precursor cells are separated from neural rosettes differentiated from pluripotent stem cells.

According to the present invention, the PSA-NCAM-positive neural precursor cells are separated from neural rosettes, which are differentiated from pluripotent stem cells through the stimulation of neural differentiation, using anti-PSA-NCAM-antibody.

As disclosed herein, there is provided a composition for treating ischemic disease or neuroinflammatory disease, the composition containing, as an active ingredient, a secretome of neural precursor cells.

As used herein, the term "secretome of neural precursor cells" refers to an aggregate of proteins secreted from neural precursor cells to the outside (culture medium) when the neural precursor cells are cultured.

As disclosed herein, the neural precursor cells used in the preparation of the secretome of the present disclosure are differentiated from pluripotent stem cells.

As disclosed herein, the neural precursor cells are neural precursor cells at the stage of neural rosettes formed by the differentiation of pluripotent stem cells (e.g., embryonic stem cells or induced pluripotent stem cells) into neural lineage cells.

According to an embodiment of the present invention, the neural precursor cells are PSA-NCAM-positive neural precursor cells.

As disclosed herein, the neural precursor cells are PSA-NCAM-negative neural precursor cells.

The PSA-NCAM-positive or negative neural precursor cells may be separated from neural rosettes, which are differentiated from pluripotent stem cells through stimulation of neural differentiation, using anti-PSA-NCAM-antibody.

As disclosed herein, the secretome is in a form of being contained in a cell culture liquid obtained by culturing neural precursor cells in an animal cell culture medium. That is, the composition for use according to the present invention may be contained in a culture liquid of neural precursor cells, containing a secretome of neural precursor cells.

As disclosed herein, the cell culture liquid of the neural precursor cells may be obtained by culturing the neural precursor cells in a serum-free animal cell culture medium containing insulin/transferrin/selenium (ITS) and basic fibroblast growth factor (bFGT) and then removing the cells. For the culturing, neural precursor cells that are obtained by subculturing (e.g., at least four passages) the neural precursor cells in a bFGF-containing animal cell culture medium supplemented with, for example, N2, B-27, and/or Gem21.

The removal of the cells from the culture medium may be conducted by using an ordinary cell separation method, such as centrifugation or filtration.

For the animal cell culture medium, an ordinary medium that is used for culturing neural precursor cells may be used without limitation. For example, DMEM/F12, DMEM or RPMI-1640 may be used.

As disclosed herein, the neural precursor cells are differentiated from human induced pluripotent stem cells, and the secretome of neural precursor cells include the following proteins:
Agrin, annexin A5, BSG(Basigin), biglycan, calponin-3, coactosin-like protein, cofilin-1, collagen alpha-2, cullin-3, destrin, dystroglycan, ephrin-B2, exportin-2, ezrin, fibronectin, fibulin-1, frizzled-related protein, gelatin-3 binding protein, granulins, growh/differentiation factor 11, haptoglobin, hemopexin, high mobility group protein B2, hornerin, importin-9, insulin-like grwoth factor-binding protein 2, Lupus La protein, macrophage migration inhibitory factor, midkine, moesin, neuropilin 2, pleiotrophin, profilin-1, protein DJ-1, radixin, secreted frizzled-related protein-2, septin-11, talin-1, testican, thymopoietin, transgelin-3 and vimentin.

As disclosed herein, the neural precursor cells are differentiated from human embryonic stem cells, and the secretome of neural precursor cells include the following proteins:
Agrin, annexin A2, attractin, biglycan, ceruloplasmin, cofilin-1, collagen alpha-1, coronin-1X, dermicidin, DERP12, eprin-B3, exostosin-2, ezrin, gelatin-3 binding protein, granulins, growh/differentiation factor 11, haptoglobin, hemopexin, high mobility group protein B2, hornerin, insulin-like grwoth factor-binding protein 2, Lupus La protein, midkine, moesin, multiple epidermal growth factor-like domains protein 8, nidogen-1, parathymosin, profilin-2, protein DJ-1, secreted frizzled-related protein-2, secretogranin, talin-1, thymosin beta-4, TGFBI(Transforming grwowth factor-beta-induced protein ig-h3), transgelin and vimentin.

Hereinafter, the common contents of the composition for use according to the present invention are described.

As used herein, the term "stem cells" is a generic term for undifferentiated cells before differentiation into respective cells constituting tissues, and the stem cells have an ability to be differentiated into particular cells by particular differentiation stimulations (environment). Unlike cell division-ceased undifferentiated cells, the stem cells are capable of producing the same cells as their own through cell division (self-renewal), and have plasticity in differentiation, in which the stem cells are differentiated into particular cells by the application of the differentiation stimulation and may be differentiated into various cells by different environments or by different differentiation.

The stem cells used in the present invention are pluripotent stem cells that proliferate indefinitely *in vitro* and can be differentiated into various cells derived from all embryonic layers (ectoderm, mesoderm, and endoderm). More specifically, the pluripotent stem cells are embryonic stem cells, induced pluripotent stem cells (iPSCs), embryonic germ cells, or embryonic carcinoma cells.

Although not part of the present invention, the embryonic stem cells are derived from the inner cell mass (ICM) of the blastocyst, and the embryonic germ cells are derived from primordial germ cells present in 5-10 week-old gonadal ridges.

Induced pluripotent stem cells (iPSCs) are one type of pluripotent stem cells artificially derived from non-pluripotent cells (e.g., somatic cells) by inserting a particular gene imparting pluripotency therein. Induced pluripotent stem cells are considered to be the same as pluripotent stem cells (e.g., embryonic stem cells) since the induced pluripotent stem cells have highly similar stem cell genes and protein expression, chromosomal methylation pattern, doubling time, embryoid body formation capacity, teratoma formation capacity, viable chimera formation capacity, hybridizability, and differentiation ability as embryonic stem cells.

The term "neural rosettes" refers to neural stems cell at the initial stage in the neural differentiation procedure of human embryonic stem cells, and the neural rosette has a cylindrical radial form. The neural rosettes are composed of cells expressing initial neuroectodermal markers, such as Pax6 and Sox1, and may be differentiated into various neural cells and neuroglial cells. The stimulation of neural differentiation may be differentiated by a method that is ordinarily conducted in the art, for example, serum-free media (Tropepe V et al., Neuron. 30:6578(2001)), fibroblast growth factors (FGFs), and treatment with morphogens, such as Wnt and retinoic acid (RA) (Ying QL et al. Nat Biotechnol. 21:183186(2003)).

Polyclonal antibodies or monoclonal antibodies may be used as the antibody. The antibodies against PSA-NCAM may be produced by the methods that are conventionally conducted in the art, for example, a fusion method (Kohler and Milstein, European Journal of Immunology, 6:511-519 (1976)), a recombinant DNA method (USP 4,816,56), or a phage antibody library method (Clackson et al, Nature, 352:624-628 (1991) and Marks et al, J. Mol. Biol., 222:58, 1-597 (1991)). A general procedure for antibody production is described in detail in Harlow, E. and Lane, D., Using Antibodies: A Laboratory Manual, Cold Spring Harbor Press, New York, 1999; Zola, H., Monoclonal Antibodies: A Manual of Techniques, CRC Press, Inc., Boca Raton, Florida, 1984; and Coligan, CURRENT PROTOCOLS IN IMMUNOLOGY, Wiley/Greene, NY, 1991. For example, hybridoma cells producing monoclonal antibodies may be obtained by fusing immortal cell lines to antibody-producing lymphocytes, the technology for which has been well known to those skilled in the art, and can be easily conducted. The polyclonal antibodies may be obtained by injecting PSA-NCAM antigens into an appropriate animal, collecting antisera from the animal, and then isolating antibodies from the antisera using the known affinity technique.

As used herein to recite the PSA-NCAM, the term "antibody" refers to a an antibody specific to PSA-NCAM, and the antibody specifically binds to the PSA-NCAM protein, and includes a complete form of an antibody and an antigen binding fragment of the antibody molecule. The complete antibody has a structure having two full-length light chains and two full-length heavy chains, and the light chains are linked to the heavy chains via a disulfide linkage, respectively. The antigen-binding fragment of the antibody molecule is a fragment having an antigen binding function, and includes Fab, F(ab'), F(ab')2, and Fv.

For the separation of PSA-NCAM-positive neural precursor cells using an antibody, fluorescence-activating cell sorters (FACS), magnetic activated cell sorter (MACS), antibody-coated plastic adherence, and complement-mediated lysis may be used.

As used herein, the term "treatment" refers to: (a) suppressing the development of disease, disorder, or symptom; (b) reducing disease, disorder, or symptom; or (c) removing the symptoms of disease, disorder, or symptom. The composition for use according to the present invention suppresses the development of symptoms of ischemic disease, or removes or reduces the symptoms of ischemic disease. Therefore, the composition of the present invention *per se* may be a composition for use in the treatment of ischemic disease. As used herein, the term "treatment" or "treatment agent" includes a meaning of "treatment aid" or "therapeutic adjuvant".

As used herein, the term "ischemic disease" refers to a disease of tissue necrosis through a reduction in the blood flow rate and a blockage of blood supply, caused by blood leakage, embolism, or infarction due to injuries of blood vessels.

According to an embodiment of the present invention, the ischemic disease that can be treated by the composition of the present invention is selected from the group consisting of ischemic heart disease, myocardial infarction, angina pectoris, lower limb artery ischemic disease, distal limb ischemic disease, and cerebrovascular ischemic disease.

As used herein, the term "ischemic heart disease" refers to a disease caused by the reduction in blood flow into heart muscle due to the damage, narrowness, and occlusion of coronary arteries for supplying blood to the heart. More specifically, the ischemic heart disease that can be treated by the composition of the present invention is selected from the group consisting of angina, myocardial infarction, and cardiac failure.

As used herein, the term "ischemic cerebrovascular disease" refers to a disease of the damage of brain tissues caused by the non-supply of blood flow due to the damage, narrowness, or occlusion of the brain blood vessels. More specifically, the ischemic cerebrovascular disease is ischemic stroke.

As used herein, the term "neuroinflammatory disease" refers to a disease caused by the damage of neural tissues due to inflammatory responses.

As disclosed herein, the neuroinflammatory disease that can be treated by the composition of the present invention is selected from the group consisting of Alzheimer's disease, Parkinson's disease, Huntington's disease, Lou Gehrig's disease, Creutzfeldt Jakob disease, multiple sclerosis, amyotrophic lateral sclerosis, diffuse Lewy body disease, leukencephalitis, temporal lobe epilepsy, and inflammatory spinal cord injury.

As used herein, the term "administration" or "administer" refers to a method wherein a therapeutically effective amount of the composition of the present invention is directly administered to a subject to form the same amount thereof in the body of the subject. Therefore, the term "administer" includes the injection of an active ingredient (a secretome of PSA-NCAM-positive neural precursor cells or neural precursor cells) around a site of lesion, and thus the term "administer" is used in the same meaning as the term "inject".

The term "therapeutically effective amount" of the composition refers to the content of an extract, which is sufficient to provide a therapeutic or prophylactic effect to a subject to be administered, and thus the term has a meaning including "prophylactically effective amount". As used herein, the term "subject" includes, human, mouse, rat, guinea pig, dog, cat, horse, cow, pig, monkey, chimpanzee, beaver, or rhesus monkey. Specifically, the subject of the present invention is human.

In cases where the composition for use according to the present invention is prepared as a pharmaceutical composition, the pharmaceutical composition of the present disclosure contains a pharmaceutically acceptable carrier. The pharmaceutically acceptable carrier contained in the pharmaceutical composition of the present disclosure is one that is conventionally used in the formulation, and examples thereof may include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, saline, phosphate buffered saline (PBS), and media.

The pharmaceutical composition of the present disclosure may further contain, in addition to the above ingredients, a lubricant, a wetting agent, a sweetening agent, a flavoring agent, an emulsifier, a suspending agent, a preservative, and the like. Suitable pharmaceutically acceptable carriers and preparations are described in detail in Remington's Pharmaceutical Sciences (19th ed., 1995).

The pharmaceutical composition of the present disclosure may be administered orally or parenterly, and examples of parenteral administration may include intramuscular administration, intracerebroventricular administration, intrathecal administration, or intravascular administration.

A suitable dose of the pharmaceutical composition of the present disclosure may vary depending on various factors, such as the method for formulation, the manner of administration, the age, body weight, gender, morbidity, and diet of the patient, time of administration, route of administration, excretion rate, and response sensitivity. The general dose of the pharmaceutical composition of the present disclosure is $10^2$-$10^{10}$ cells per day on the basis of an adult.

The pharmaceutical composition of the present disclosure may be formulated into a unit dosage form or may be prepared in a multi-dose container by using a pharmaceutically acceptable carrier and/or excipient according to the method easily conducted by a person having an ordinary skill in the art to which the present invention pertains. Here, the dosage form may be a solution in an oily or aqueous medium, a suspension, a syrup, or an emulsion, an extract, a pulvis, a powder, a granule, a tablet, or a capsule, and may further include a dispersant or a stabilizer.

Advantageous Effects

Features and advantages of the present invention are summarized as follows:

(a) The present invention provides a composition for use in the treatment of ischemic disease
(b) PSA-NCAM-positive neural progenitor cells used in the present invention promote angiogenesis in injected tissue and inhibit an inflammatory response. The PSA-NCAM-positive neural progenitor cells can be simply isolated by using an anti-PSA-NCAM-antibody, and exhibit excellent angiogenic and anti-inflammatory activities compared with mesenchymal stem cells, and thus can be useful as a composition for effectively treating ischemic diseases caused by a vascular injury and nerve damage diseases caused by inflammation.
(c) Although not part of the present invention, a secretome of the neural progenitor cells of the present disclosure reduces the ischemic injury site and allows a neurological function to recover, and thus can be used as an agent for treating ischemic diseases and degenerative nervous system disorders such as nerve damage diseases caused by inflammation.

Brief Description of the Drawings

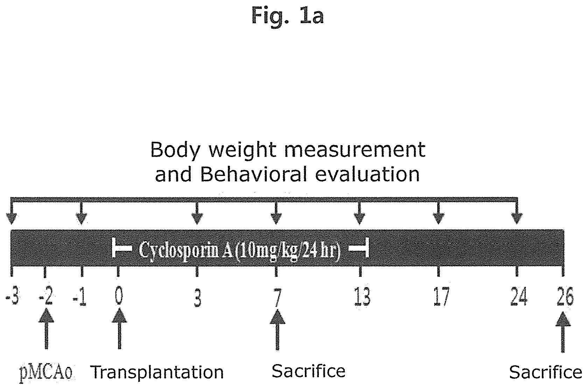

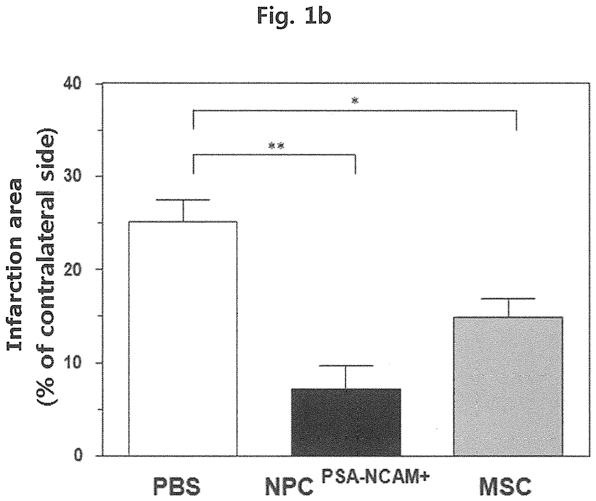

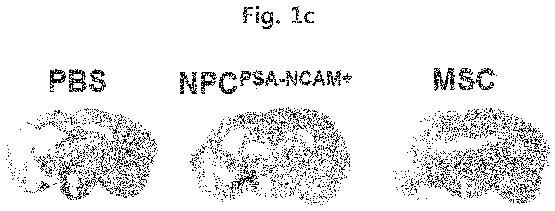

FIG. 1 illustrates the shrinkage of infraction area in rat ischemic brain transplanted with NPC$^{PSA-NCAM+}$ and MSCs. FIG. 1a is a schematic view showing the experimental design. One day after pMCAo, animals were randomly allocated to receive NPC$^{PSA-NCAM+}$, MSCs, or PBS (n=10 each). At day 26 following transplantation, animals were sacrificed, and the brains were processed for immunohistochemistry. FIG. 1b shows effect of MSCs and NPC$^{PSA-NCAM+}$ transplantation on infarction size in a rat model with pMCAo at day 26 post-transplantation. *P<0.05 and **P<0.01 when compared with PBS group. FIG. 1c shows representative images obtained at day 26 after cell transplantation in rats treated with PBS, NPC$^{PSA-NCAM+}$, or MSCs.

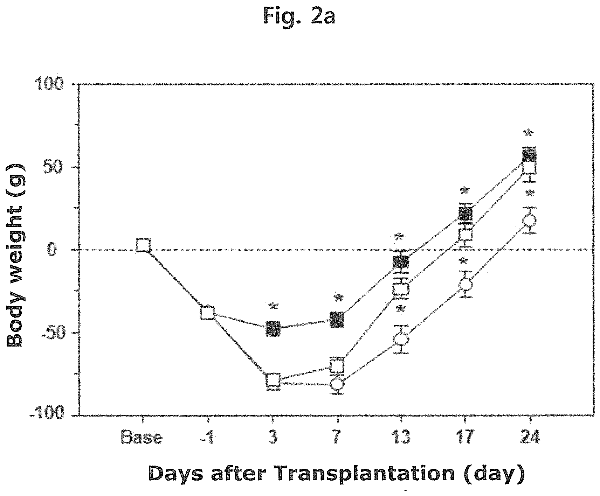

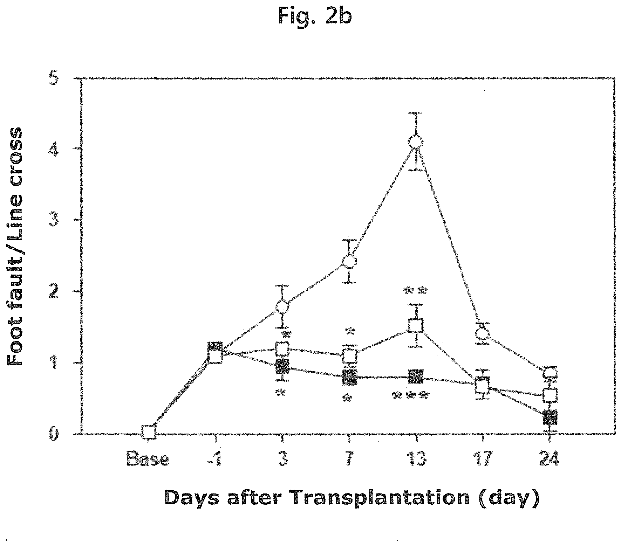

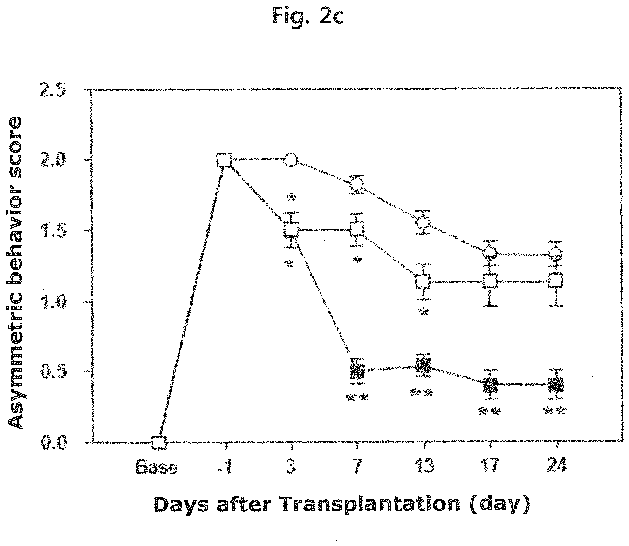

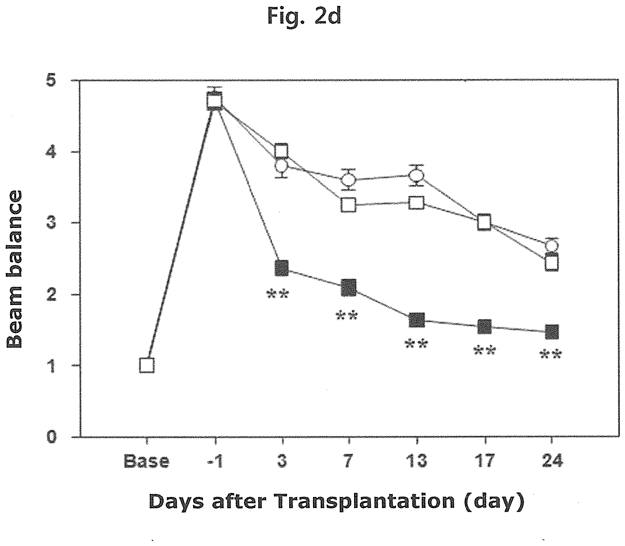

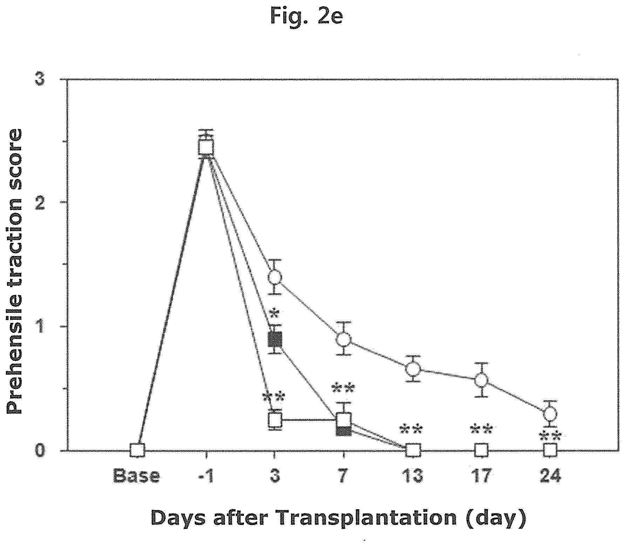

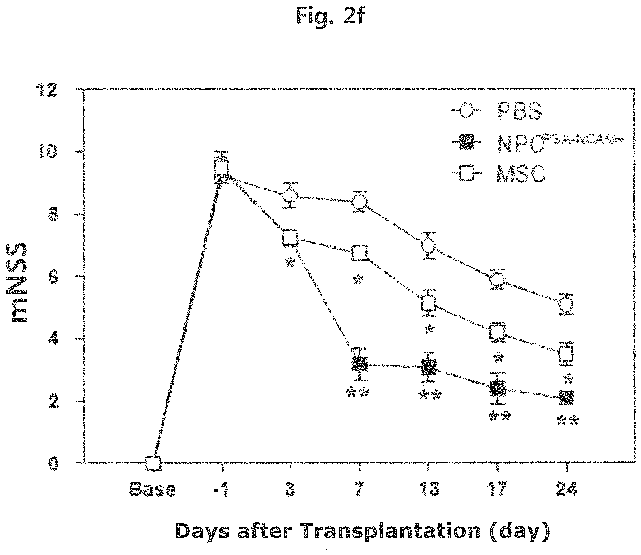

FIG. 2 illustrates enhancement of behavioral performance in rat stroke model transplanted with NPC$^{PSA-NCAM+}$. Respective images show results of body weight change (FIG. 2a), foot fault test (FIG. 2b), asymmetric test (FIG. 2c), beam balance test (FIG 2d), prehensile traction test (FIG. 2e), and mNSS (FIG. 2f). Measurement values are mean±S.E.M. *P<0.05, **P<0.01, and ***P<0.001 when compared with PBS group.

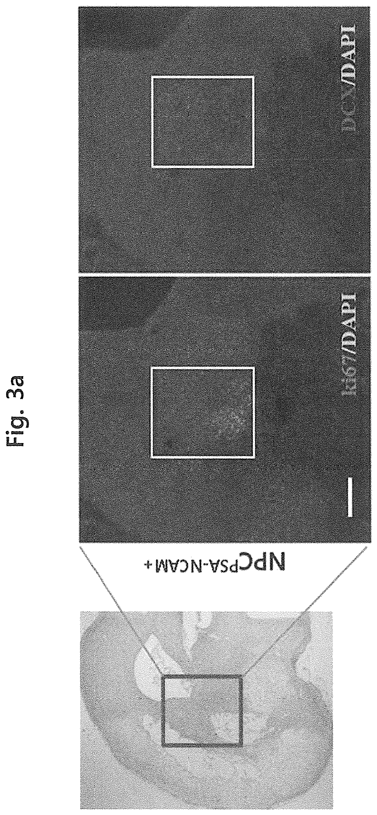

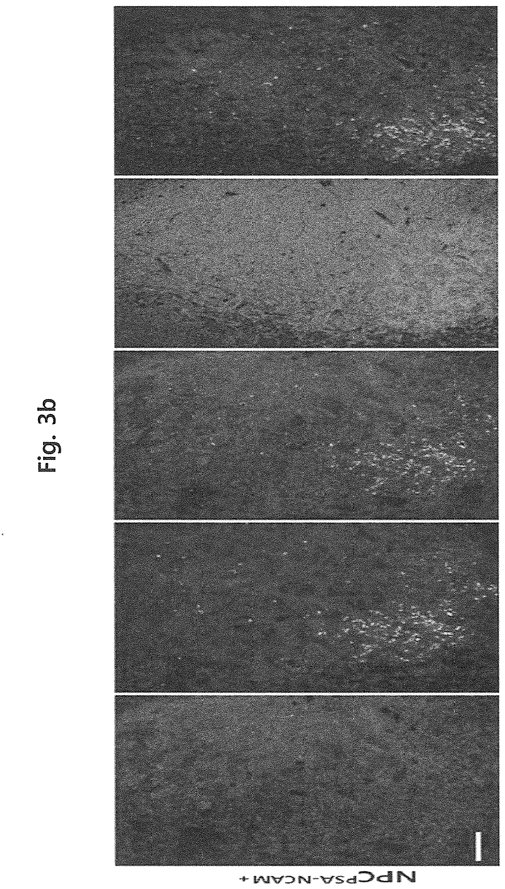

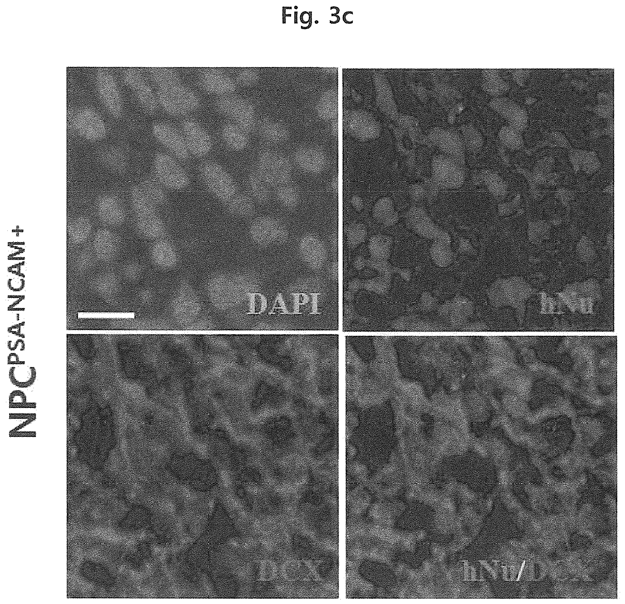

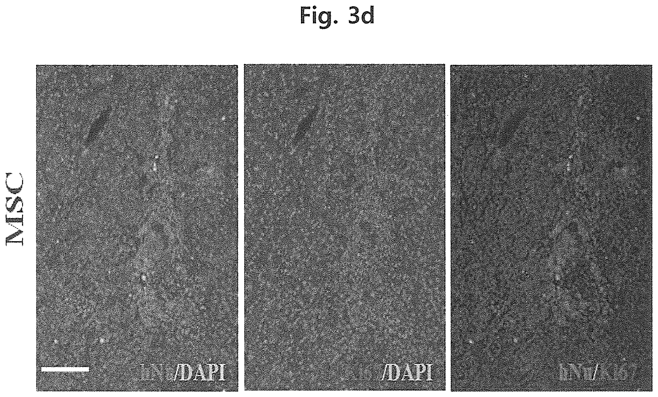

FIG. 3 illustrates integration and differentiation of viable NPC$^{PSA-NCAM+}$ into rat brain tissue. FIG. 3a shows an infraction site. Survival and proliferation of transplanted NPC$^{PSA-NCAM+}$ were tested at day 26. Ki67 (green) or DCX (red) positive cells were observed in rat ischemic brain. Scale bar: 500 μm. FIG. 3b shows high magnifications of the inset in FIG. 3a. Scale bars: 200 μm. FIG. 3c shows examples of NPC$^{PSA-NCAM+}$ cells co-expressing hNu (green) and DCX (red). Many grafted NPC$^{PSA-NCAM+}$ cells demonstrated hNu positivity, indicating good survival, and integration into damaged brain. Scale bars: 20 μm. FIG. 3d shows that few proliferating Ki67+ hNu- cells were observed in and around the needle tract in most cases of MSC-grafted groups. Scale bars: 200 μm. FIG. 3b gives images with respect to DCX/DAPI, Ki67/DAPI, DCX/Ki67/DAPI, Tuj1/DAPI, and Nestin/Ki67/DAPI, from the left side. FIG. 3c gives images with respect to DAPI, hNu, hNU/DCX, and DCX in a clockwise direction from the left. FIG. 3d gives images with respect to hNU/DAPI, Ki37/DAPI, and hNU/Ki67 from the left side.

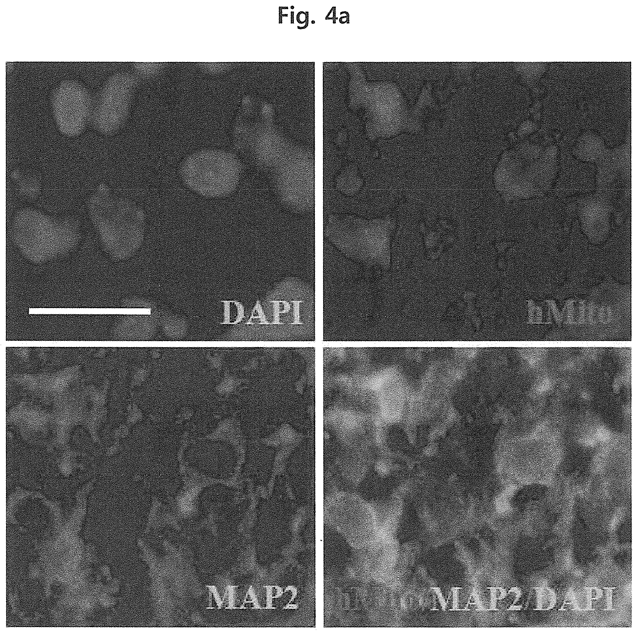

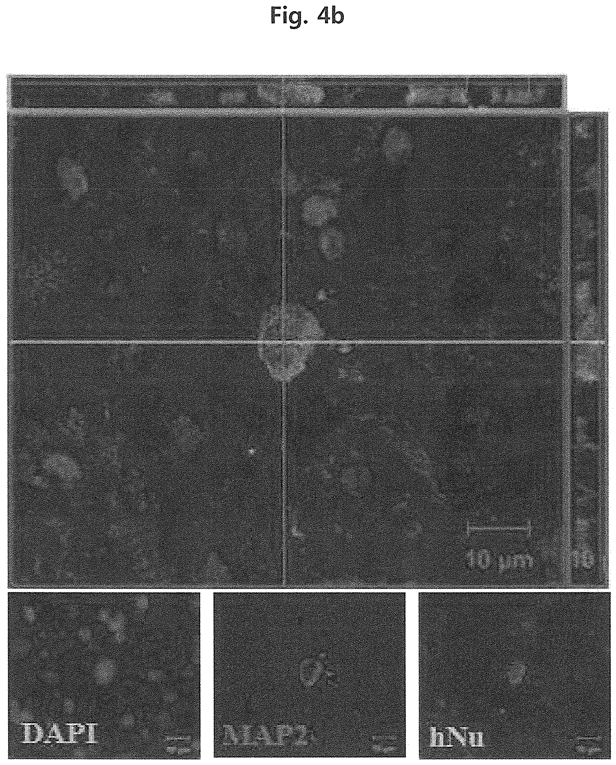

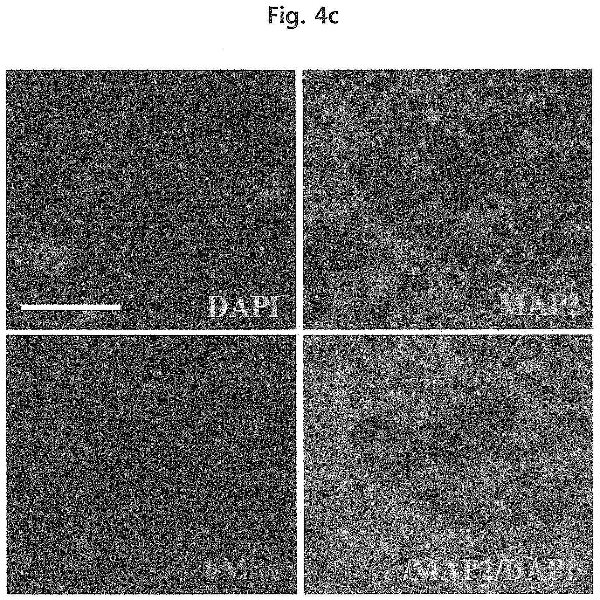

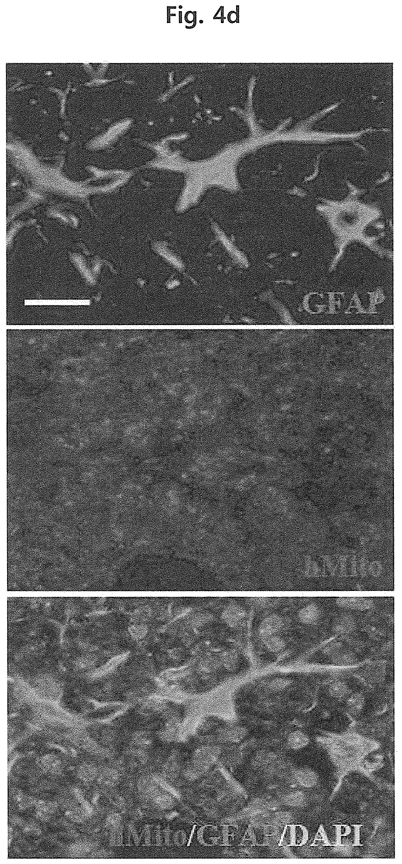

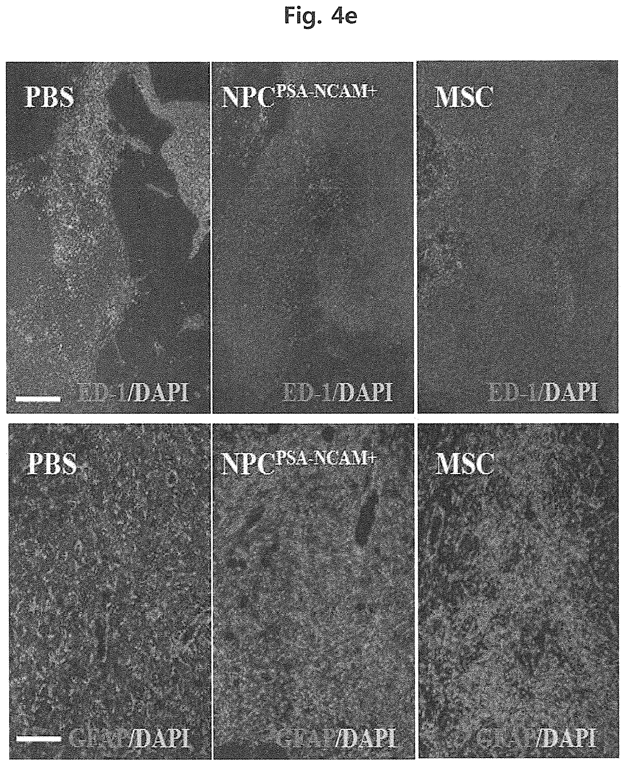

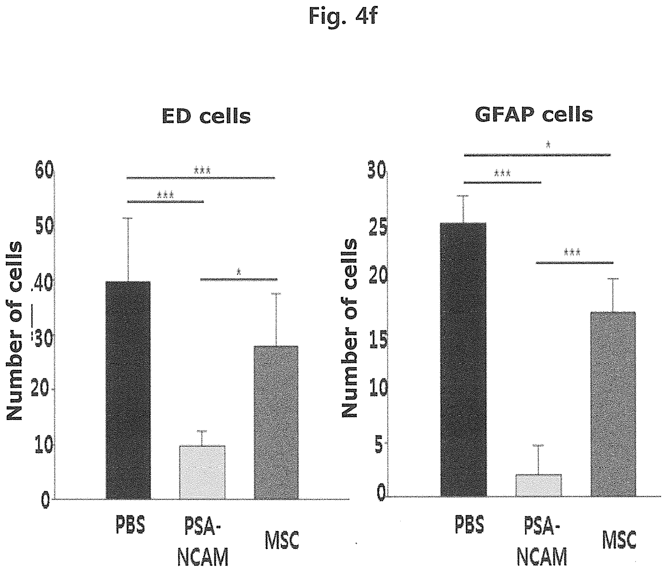

FIG. 4 illustrates neuronal commitment of NPC$^{PSA-NCAM+}$ and reduction of glial activation in rat brain tissue. FIG. 4a shows that cells with hMito+ (red) and MAP2+ (green) were observed in NPC$^{PSA-NCAM+}$-transplanted rat brain. Scale bar: 20 μm. FIG. 4b shows confocal images of grafted cells at day 26 after transplantation. The donor-derived cells (green, hNu+) co-localized with MAP2 (red) within the grafts generated by NPC$^{PSA-NCAM+}$ transplantation. DAPI, blue. FIG. 4c shows host neurons in the contralateral striatum were devoid of hMito co-localized with MAP2. Note the prominent MAP2+ cells in the striatum. Scale bar: 20 μm. FIG. 4d shows that the hMito+GFAP+ cells were not detectable in the NPC$^{PSA-NCAM+}$-transplanted rat brain. Scale bar: 20 μm. FIG. 4e shows ED-1 positivity in the ipsilateral striatum was significantly reduced in NPC$^{PSA-NCAM+}$ -transplanted group, and to a lesser extent in MSC-transplanted group. Both NPC$^{PSA-NCAM+}$- and MSC-transplanted groups were significantly different from PBS group. Expression of GFAP was notably lower in NPC$^{PSA-NCAM+}$-transplanted group than in MSC- or PBS groups. Scale bar: 200 μm. FIG. 4f shows the number of ED1 or GFAP positive cells counted in at least five separate microscopic fields. Measurement values are mean±S.E.M. *P<0.05 and ***P<0.001 when the multiple comparison were made among the groups.

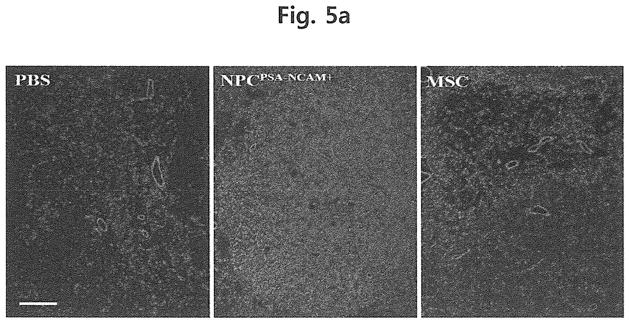

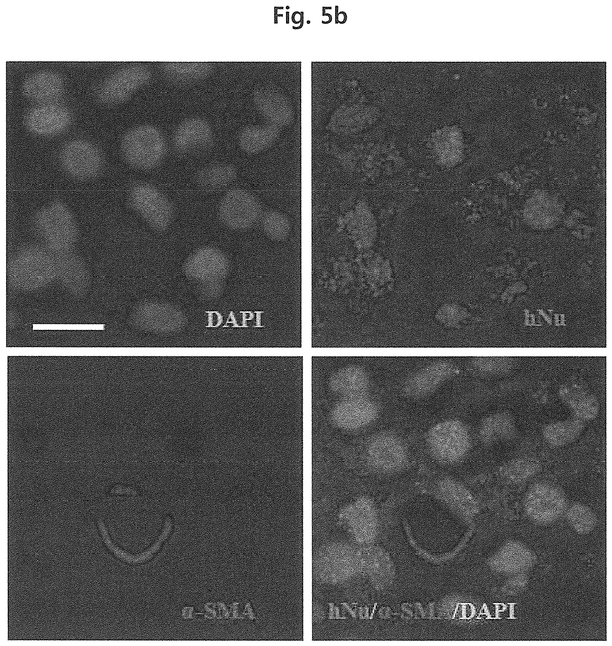

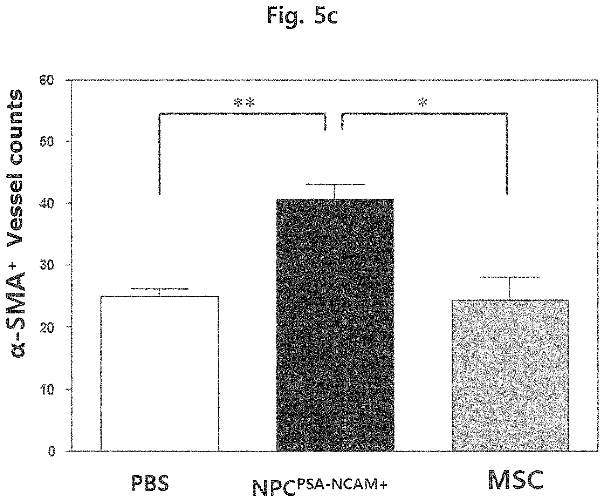

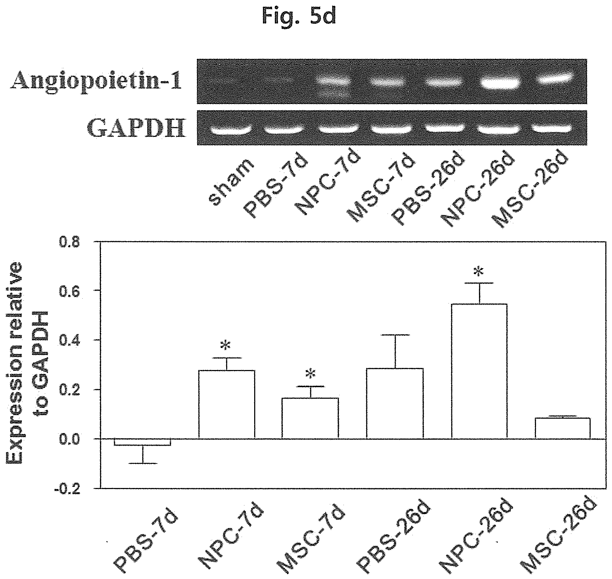

FIG. 5 illustrates the stimulation of angiogenesis in rat ischemic brain transplanted with NPC$^{PSA-NCAM+}$. FIG. 5a shows immunostaining results of ischemic brain of PBS group, NPC$^{PSA-NCAM+}$- or MSC-transplanted group, respectively. Scale bar: 200 μm. FIG. 5b shows representative images of host origin α-SMA-positive vascular endothelial cells (red) at the NPC$^{PSA-NCAM+}$-grafted region. Scale bar: 20 μm. FIG. 5c shows quantitative analysis results of α-SMA-positive microvessels in NPC$^{PSA-NCAM+}$- or MSC-transplanted rats. Measurement values are mean±S.E.M. *P<0.05 and P<0.01 in NPC$^{PSA-NCAM+}$ group when compared with MSC or PBS group, respectively. FIG. 5d shows results of the expression levels of rat angiopoietin-1 in ischemic brain assessed using RT-PCR at day 7 and 26 after transplantation with NPC$^{PSA-NCAM+}$ (NPC) or MSCs. The RT-PCR amplification of angiopoietin-1 (top) and quantification of GAPDH-normalized mRNA levels to that of sham control (baseline) (bottom) are shown (n=3 per group) are shown. Measurement values are mean±S.E.M. P<0.05 when compared with PBS group.

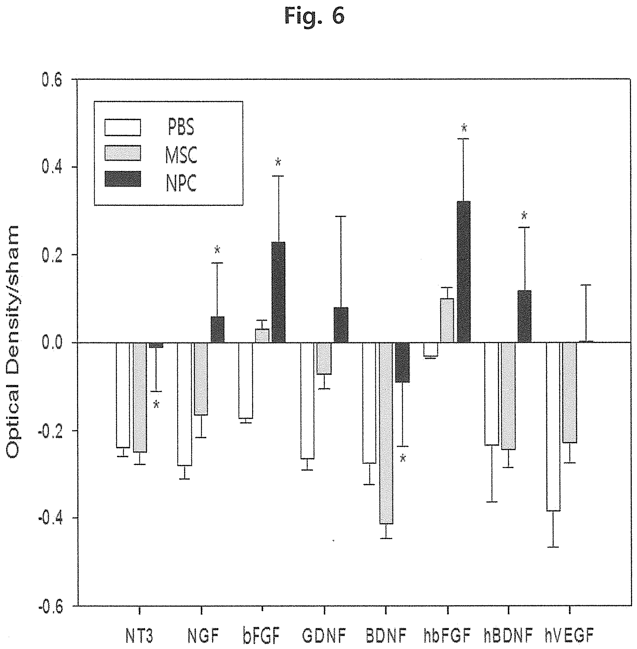

FIG. 6 illustrates the expression levels of rat and human neurotrophic factors in ischemic brain, assessed using RT-PCR at day 26 after transplantation with NPC$^{PSA-NCAM+}$, MSCs, or PBS. The RT-PCR amplification of neurotrophic factors and the quantification of GAPDH-normalized mRNA levels to that of sham controls (baseline) (n=3 per group) are shown. Measurement values are mean±S.E.M. *P<0.05 when compared with PBS group.

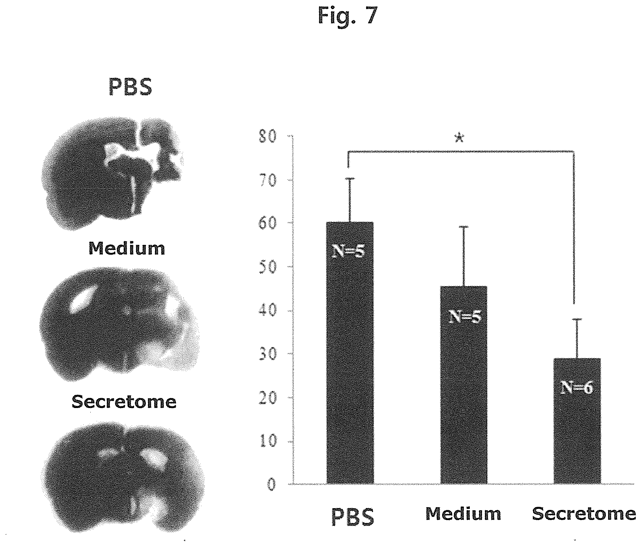

FIG. 7 shows sizes of ischemic lesion site in PBS control group, medium control group, and secretome-treated group.

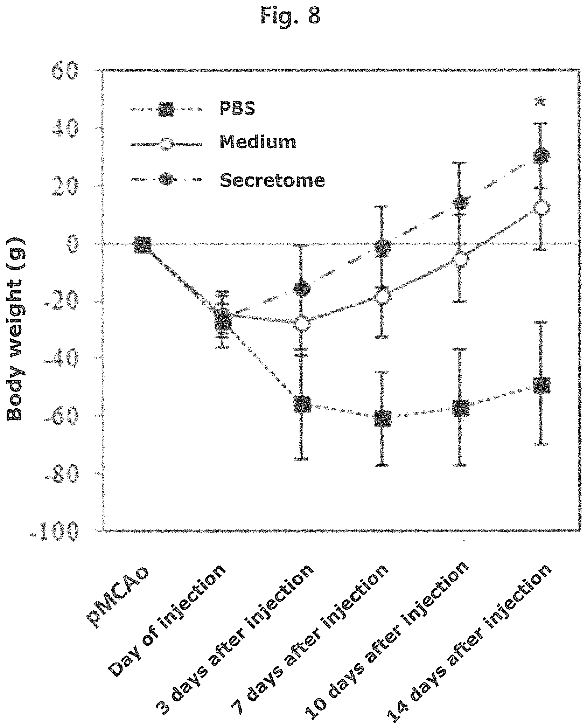

FIG. 8 illustrates the change in body weight in the PBS control group, medium control group, and secretome-treated group.

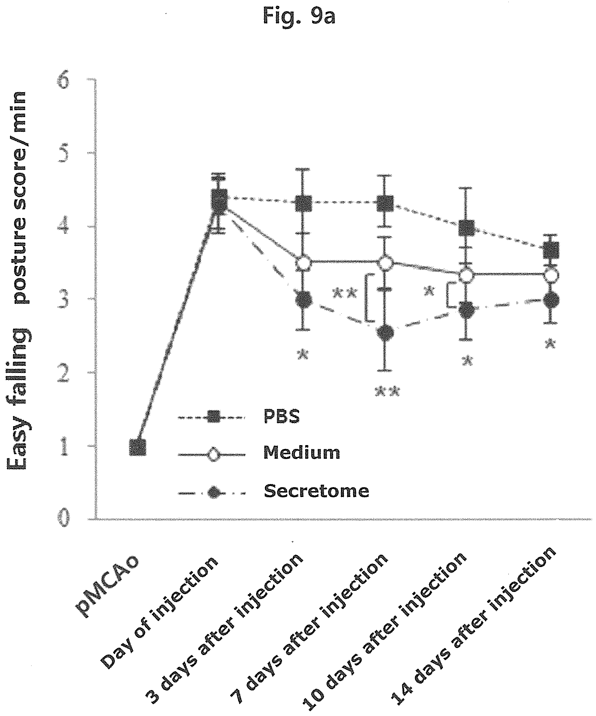

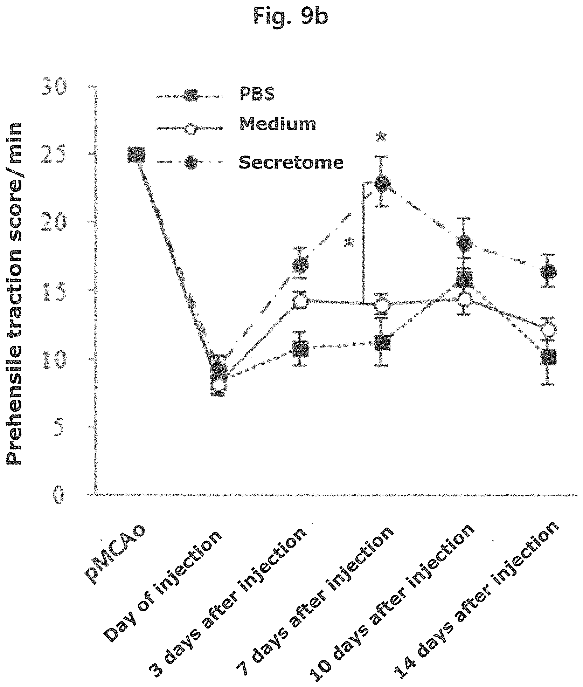

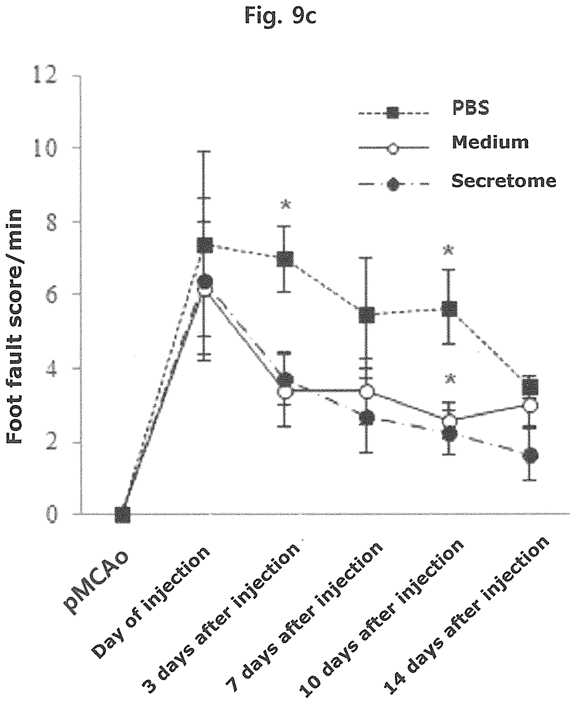

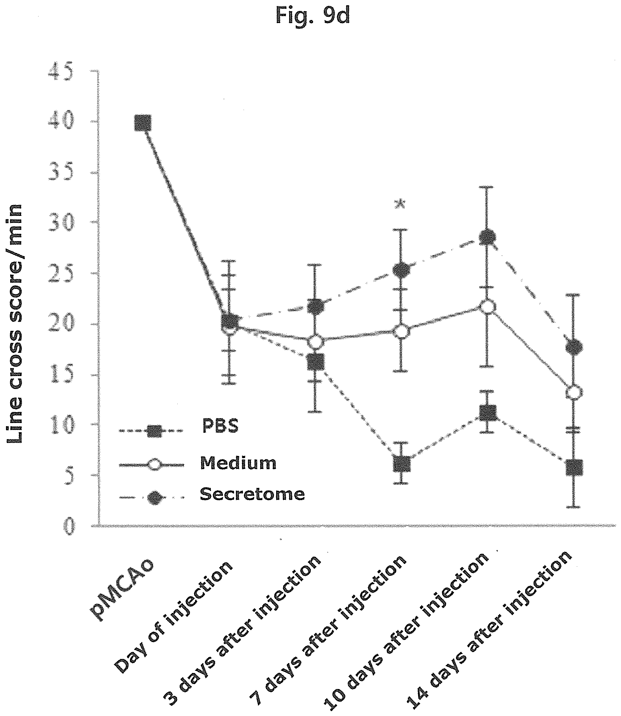

FIG. 9 illustrates behavior analysis results in the PBS control group, medium control group, and secretome-treated group. FIG. 9a shows beam balance test result; FIG. 9b shows prehensile traction test results; FIG. 9c shows foot fault test results; FIG. 9d shows line cross results indicating the activeness of behavior per unit time.

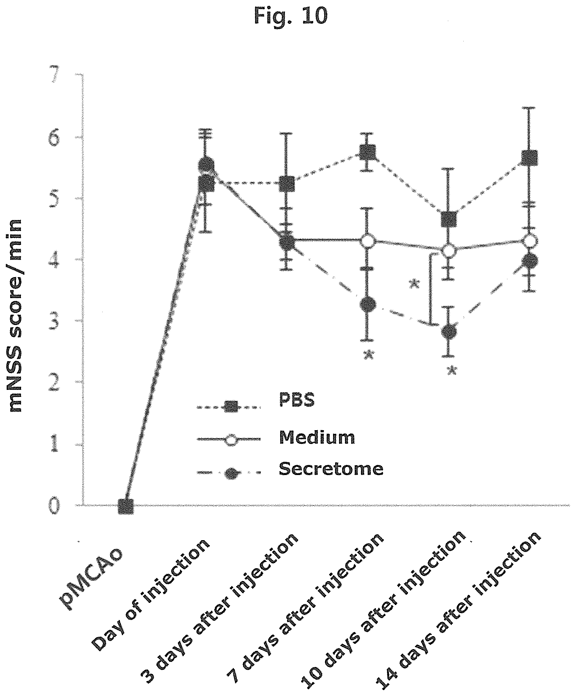

FIG. 10 shows comprehensive behavioral neuron improvement effect (mNSS) analysis results in PBS control group, medium control group, and secretome-treated group.

*P value < 0.05, **P value < 0.01 in FIGS. 7 to 10.

Mode for Carrying Out the Invention

Hereinafter, the present invention will be described in detail with reference to examples.

**EXAMPLES**

**Example 1: Treatment Effect of PSA-NCAM-positive Nural Precursor Cells On Ischemic Disease And Neuroinflammatory Disease**

**Methods**

*Culture and Differentiation of Human MSCs and Human ESC-Derived NPC$^{PSA-NCAM+}$*

The use of human cells was approved by the Institutional Review Board (IRB No. 4-2008-0643). Human bone marrow was obtained by aspiration from the posterior iliac crest from healthy adult volunteers who provided informed consent. Briefly, bone marrow mononuclear cells were isolated using density gradient centrifugation (GE Healthcare, Uppsala, Sweden) and were plated at a density of $1\times106$ cells/cm2 in DMEM supplemented with 10 % FBS (Gibco, Grand Island, NY), and cultured at 37□ in a humidified atmosphere containing 5 % $CO_2$. After 24 h, non-adherent cells were washed and removed. The medium was changed every 3rd day and the cells were sub-cultured using 0.05 % trypsin/EDTA (Invitrogen, Carlsbad, CA) when they reached 90 % confluence.

Adherent MSCs at passages 3-5 were used for this study. For neural induction, embryoid bodies (EBs) derived from hESCs were cultured for 4 days in suspension with 5 μM dorsomorphin (DM) (Sigma, St. Louis, MO) and 5-10 μM SB431542 (SB) (Calbiochem, San Diego, CA) in hESC medium deprived of bFGF (Invitrogen), and then attached on Matrigel-coated dishes (BD Biosciences, Bedford, MA) in $1\times$N2 (Invitrogen) media supplemented with 20 ng/ml bFGF for the additional 5 days (Kim, D. S., Lee, D. R., Kim, H. S., et al. (2012). *PLoSOne,* 7, e39715). Neural rosettes that appeared in the center of attached EB colonies were carefully isolated using pulled glass pipettes from the surrounding flat cells. Small rosette clumps were then seeded on Matrigel-coated dishes after gentle trituration and cultured in DMEM/F12 supplemented with $1\times$ N2, $1\times$ B27 (all from Invitrogen) (referred to as N2B27 medium) plus 20 ng/ml bFGF (Kim, D. S., Lee, J. S., Leem, J. W., et al. (2010). Stem Cell Reviews and Reports, 6, 270-281).

*Isolation of PSA-NCAM-Positive NPCs by MACS*

Expanded neural rosette cells at 80-90 % confluence were exposed to 10 μM Y27632 (Sigma) for 1 h to prevent cell death prior to being subjected to MACS procedure. After dissociation using Accutase (Invitrogen), the cells (~$1\times10^8$ cells) were briefly blocked in PBS with 2 % BSA, and then incubated with anti-PSANCAM antibody conjugated with microbeads (Miltenyi Biotec) for 15 min at 4□. After extensive washing, the cell suspension was subjected to the MACS procedure and positively-labeled cells that remained in the column were eluted to a tube with culture media. Isolated NPC$^{PSA-NCAM+}$ were re-plated on the culture dish at a density of $4-5\times10^5$ cells/cm$^2$ in N2B27 medium or NBG medium plus 20 ng/ml of bFGF ($1\times$ N2, $0.5\times$ B27, and $0.5\times$ G21 supplement (Gemini BioProducts, West Sacramento, CA)). Culture medium was changed every day and the cells were passaged every 2-3 days.

*Establishment of Stroke Model and Stereotaxic Injection of NPC$^{PSA-NCAM+}$*

The 2-month aged male Sprague-Dawley rats (approximately 250-300 g in body weight) were anesthetized with 3 % isoflurane (Hana Pharm, Seoul, Korea) in a 70-30 % mixture of $N_2O$ to $O_2$. The left common carotid artery and external carotid artery were isolated and ligated with a 4-0 surgical suture. A nylon thread was inserted into the left internal carotid artery and advanced to the Circle of Willis (permanent middle cerebral artery occlusion: pMCAo). The thread was left in place until the rats were sacrificed.

Stereotaxic injection of NPC$^{PSA-NCAM+}$, MSCs, or PBS was performed 2 days after pMCAo. Rats were anesthetized with zoletil (Virbac S.A., France, 25 mg/kg) and then placed in a stereotaxic surgical apparatus (David Kopf Instruments, Tujunga, CA). A 26-gauge needle (Hamilton syringe, Hamilton, Reno, NV) was inserted into the left striatum (coordinates from the bregma: anteroposterior +0.7 mm, mediolateral -2 mm and dorsoventral -5.5 mm, - 2.5 mm from meninges); 5 μl of NPC$^{PSA-NCAM+}$ or MSCs ($1\times105$ cells/μl each) or PBS was injected into the 5.5 mm and - 2.5 mm sites. All cells were constantly infused into the left striatum at a rate of 1 μl/min with continuous agitation to prevent cellular aggregation. Nine animals died while pMCAo surgery and cell transplantation were performed.

All animals were housed in the facility approved by the Association for Assessment and Accreditation of Laboratory Animal Care (AAALAC), using a 12 h light/dark cycle. The experiment was approved by the Institutional Review Board (IRB No. 4-2011-0087).

*Behavioral Tests*

Foot fault test: The foot fault test measures the accuracy of forepaw placement on an equi-distant grid (60×60 cm, 6 cm distance) during a 2-min testing period. This test was a modified procedure that is based on the previous study [12, 13].

Asymmetric behavior test: The modified elevated body swing test was adapted from the previous report. Rats were examined for lateral movements after elevation by their tails up to 10 cm above the surface of the testing area. The frequency of left or right swing was scored for 1 min. Asymmetric score value was calculated by the following scores; score 0-torso twist, left and right swing, score 1-asymmetric twist<30°, score 2-asymmetric twist>30°. According to the direction of torso twisting, the score was counted as either ipsilateral twist (same as infarct site) or contralateral twist (opposite of infarct site).

Beam balance test: The beam walking apparatus is composed a beam (100×5×2 cm). Motor performance was graded on a 6-point scale adapted from a previous description; score 1-balance with steady posture and paws on top of the beam, score 2-grasps side of beam and has shaky movement, score 3-one or more paws slip off beam, score 4-attempts to balance on the beam but falls off, score 5-drapes over the beam but falls off, score 6-falls off the beam with no attempt to balance.

Prehensile traction test: The prehensile portion of the test involves the rat's ability to hang onto the horizontal rope by its forepaws. The prehensile traction test was used to measure the rat's muscle strength. This test was adapted from similar tests described previously. The steel bar (2-cm diameter, 100-cm length) was placed horizontally 70 cm above the sponge rubber pad (7.5 cm thickness). The rat's forepaws were placed on the steel bar and the animal was released. The rat was allowed to hang onto the steel bar for up to 5 s. Time of falling was noted as well as whether or not rat brought the rear limb up to the bar with the following scores; score 0-the rat hangs on for 5 s and brings rear limb up, score 1- rat hangs on for 5 s and no rear limb is brought up, score 2- rat hangs on for 3-4 s, score 3-the rat hangs on for 0-2 s.

Modified neurological severity score (mNSS): The neurological severity score is a composite of motor, sensory, and reflex tests as described previously. The objective quantifications were based on the asymmetric behavior test, beam balance test, prehensile traction test, open field test (rotation frequency), and foot fault test with the following scores; score 0-no deficits, score 2-difficulty in fully extending the contralateral forelimb ($3 \leq$ front foot fault<10), score 4-unable to extend the contralateral forelimb (front foot fault$\geq$10), score 6-mild circling to the contralateral side ($1 \leq$ rotation or asymmetric twisting<5), score 8-severe circling (rotation or asymmetric twisting$\geq$5), score 10-falling to the contralateral side (prehensile traction$\leq$2).

*Immunohistochemical Analysis and Quantification*

The brain was fixed for 24 h in 4 % paraformaldehyde and washed with PBS. For the paraffin sections, the tissues were dehydrated in a graded ethanol series and then embedded in paraffin. The paraffin-embedded brain was sectioned into 5-$\mu$m-thick layers on a microtome, deparaffinized in xylene for 10 min, and rehydrated in a graded alcohol series. Sections were treated with 10 mM citric acid for 1 h followed by the addition of 5 % BSA solution containing PBS and 0.5 % Triton X-100. Thereafter, the brain sections were incubated with primary antibodies to DCX (Abcam), Tuj1 (Covance), hNu (Millipore, clone 235-1), GFAP (Millipore), Ki67 (Leica Microsystems), hMito (Millipore), MAP2 (Millipore), human Nestin (Millipore, clone 10C2), or ED-1 (Abcam)(1:100) for 10-12 h at 4□. Following the overnight incubation with the primary antibodies, the sections were washed with PBS before incubating them with a fluorescently-labeled secondary antibody, anti-rabbit IgG, for 31 h. The sections were then washed once more with PBS before being mounted with 1 $\mu$g/ml 4',6'-diamidino-2-phenylindole (DAPI). A fluorescent microscope (Olympus IX71) was used to produce fluorescent images of the sections. In order to accurately determine the numbers of neuronal cells in the brain that reacted with specific antibodies, a blind test was utilized. Three individuals, all of whom had no previous knowledge of the experiment, were asked to count the number of ED-1[+] cells and $\alpha$-SMA[+] vessels (with diameter of 50 $\mu$m or less) in five of the squares with 50 mm[2] on a slide. An investigator then took the results of all three individuals to accurately determine the number of detected neurons.

Twenty six days after transplantation, the rats (n=10 per group) were anesthetized with zoletil (Virbac S.A., France, 25 mg/kg) and perfused with PBS and 4 % paraformaldehyde in PBS (pH 7.5).

For infarct area measurement, the brain sections were stained with hematoxylin and photographed with a microscope (Zeiss, Oberkochen, Germany). The indirect lesion area, in which the intact area of the ipsilateral hemisphere was subtracted from the area of the contralatera hemisphere, was calculated. Relative infarct area was analyzed with NIH Image J program (version 1.47). It was presented as an average percentage of the indirect lesion compared with contralateral hemisphere.

*Reverse Transcription-Polymerase Chain Reaction (RT-PCR)*

Total RNA was isolated using Trizol reagent (Invitrogen). Standard reverse-transcription (RT) was first conducted using Transcriptase II (Invitrogen) and then, RT-PCR was performed with specific PCR primer set: Forward primer: TGTGTCCATCAGCTCCAGTTGC, Reverse primer: CGGCTACCATGCTCGAGATAGG) for angiopoietin 1 (Bioneer, Daejeon, Korea). The PCR products were run at 1.2 % agarose gel and then stained with ethidium bromide to detect bands (approximate size: 400 base pairs) under UV light. Finally, the detected bands were quantified by use of NIH Image J program (version 1.47).

*Statistical Analysis*

The data were expressed as mean±S.E.M. Data of behavioral test and infarct area were analyzed by ANOVA and independent t-test using the Statistical Package for Social Sciences (SPSS) version 20.0. Differences with P-values<0.05 were considered to be statistically significant.

**Results**

*Transplantation of NPC$^{PSA-NCAM+}$ Reduced Infarction Area in Host Brain*

The overall design for this experiment is shown in FIG. 1a. The severity of neural tissue damage was determined as brain area which is not normally stained with hematoxylin at day 26 following transplantation. Infarct lesions mainly appeared on the cortex and striatum. Infarction area was obviously larger in ischemic brains treated with PBS than in those transplanted with MSCs or NPC$^{PSA-NCAM+}$ (FIG. 1b). The percentage of infarction area relative to the contralateral side was significantly reduced in rats transplanted with NPC$^{PSA-NCAM+}$ (7.1±2.5 %) or MSCs (14.9±1.9%) compared to that of the PBS group (25.1±2.4%) (F=13.64, P<0.01). Although the infarction areas of NPC$^{PSA-NCAM+}$- and MSC-transplanted groups were not statistically different, NPC$^{PSA-NCAM+}$-transplanted rats obviously showed smaller infarction area than that of MSC-transplanted rats (FIG. 1c).

*Transplantation of NPC$^{PSA-NCAM+}$ Improved Behavioral Performances of A Rat Stroke Model*

The rats lost 40 g of their body weight compared to a baseline measured on the first day after pMCAo. The weight loss reached maximum at day 7 after treatment in PBS-treated rats. In MSC-transplanted group, the body weight was recovered to baseline state by day 17 after transplantation. However, the significant recovery of body weight began to appear from day 3 after transplantation in the NPC$^{PSA-NCAM+}$-transplanted group when compared with PBS controls (P<0.05) (FIG. 2a). After pMCAo, the foot faults significantly decreased in rats transplanted with NPC$^{PSA-NCAM+}$ and MSCs compared with PBS controls, from day 3 (P<0.05) through 13 (P<0.01) (FIG. 2b). Additionally, NPC$^{PSA-NCAM+}$-transplanted group showed a significant reduction in foot faults/line cross compared with MSC-transplanted group on day 13. Yet, this was less evident in later days (day 17 and 24 after transplantation) when the animals' activity decreased due to weight gain.

While all rats displayed relatively little asymmetry in EBTS before pMCAo, asymmetry was evident in all animals after pMCAo. On day 3 post-transplantation, NPC$^{PSA-NCAM+}$- and MSC-transplanted groups started to show ipsilateral twisting behavior. Whereas MSC-transplanted group showed modest improvement up to day 13 after transplantation (P<0.05), the asymmetric behavior score significantly decreased in NPC$^{PSA-NCAM+}$- transplanted group at day 7 and lasted up to day 24 after transplantation (P<0.01) (FIG. 2c).

NPC$^{PSA-NCAM+}$ transplantation also improved functional recovery in the beam balance test from day 3 through 24, compared with MSC-transplanted group and PBS controls (P<0.01) (FIG. 2d). NPC$^{PSA-NCAM+}$ transplantation significantly increased the retention time on the beam, which is well correlated with motor coordination, while no statistical difference was shown between MSC- and PBS-treated groups.

Prehensile traction score, negatively related with muscle strength of the forepaw, gradually decreased in NPC$^{PSA-NCAM+}$ -transplanted group from day 3 through 24 (P<0.01) (FIG. 2e). MSC-transplanted group also showed a similar pattern of improvement from day 7 through 24 after transplantation.

To normalize the quantifications of neurologic outcome, the present inventors finally assessed the mNSS criteria based on a asymmetric behavior score, beam balance test, prehensile traction test, open field test (rotation frequency, data not shown), and foot fault test. The mNSS showed that progressive recovery in neurological function becomes significant after day 3 in both NPC$^{PSA-NCAM+}$- and MSC-transplanted groups compared to PBS-treated groups (P<0.05) (FIG. 2f). Moreover, NPC$^{PSA-NCAM+}$- transplanted group showed substantial improvement in comparison with MSC-transplanted group from day 7 through 24 (P<0.01). The effect of NPC$^{PSA-NCAM+}$-transplanted group was especially apparent from day 3 through 7, suggesting strong paracrine effect at the early time point after transplantation.

***Transplanted NPC^PSA-NCAM+ Survived and Differentiated Into With Neuronal Commitment in Host Brain***

To track destiny of transplanted cells, the present inventors performed histologic analysis with hNu (human-specific nuclei), Ki67 (proliferating cell marker), DCX (neuroblast marker), nestin (neural stem cell marker), and Tuj1 (neuronal marker) antibodies at day 26. Following transplantation of NPC^PSA-NCAM+, most cells were found at the original transplantation site (i.e., striatum). The survival, proliferation, and differentiation of transplanted NPC^PSA-NCAM+ were confirmed by Ki67+, DCX+, Tuj1+, and nestin+ cells in damaged brain (FIGS. 3a and 3b). A majority of transplanted NPC^PSA-NCAM+ expressed either DCX or Tuj1, whereas a portion of them were positive for nestin. Mitotic state of transplanted cells was revealed by Ki67-immunoreactivity. A subset of hNu+ cells (9184 counts) was positive for Ki67 (432 cells) at day 26 following transplantation (4.7 %). Tuj-1 immunoreactivity largely overlapped with that of DCX in NPC^PSA-NCAM+ (FIG. 3b) . These DCXimmunoreactive cells were not of rat origin but of human origin (FIG. 3c), implying that the reduced brain infarction area seen with NPC^PSA-NCAM+ was due in part to transplanted cell integration.

Whereas many transplanted NPC^PSA-NCAM+ demonstrated good survival and integration into damaged tissue, only a few MSCs were detected with hNu expression at day 26 following transplantation (FIG. 3d). Most grafted NPC^PSA-NCAM+ were immature neuronal cells (i.e., DCX positive cells with a fraction of Ki67 positive cells) (FIG. 3b). In contrast, transplanted MSCs (hNu+ cells) did not exhibit DCX positivity in the lesions, and a small number of proliferating hNu-Ki67+ cells were in and around the needle tract in most cases of MSC-grafted groups (FIG. 3d).

While the hMito+MAP2+ and hNu+MAP2+ double-labeled cells were detected in NPC^PSA-NCAM+-transplanted rats (FIGS. 4a and 4b), hMito+GFAP+ (FIG. 4d) or hMito+GalC+ (data not shown) co-stained cells were hardly detectable, indicating that NPC^PSA-NCAM+ are progenitor cells with mainly neuronal commitment. No hMito+ cells were observed in contralateral striatum (FIG. 4c).

***Transplantation of NPC^PSA-NCAM+ Suppressed Reactive Glial Activation in Host Brain***

Expression of GFAP (astrocyte marker), was notably low in NPC^PSA-NCAM+-transplanted group, although some GFAP positive cells were found along the needle tracks (FIG. 4e). In fact, the number of GFAP-positive cells greatly reduced in the NPC^PSA-NCAM+ group (P<0.001), and to a lesser extent in the MSC-transplanted group (P<0.05) compared to that of PBS group (FIG. 4e, f). These findings suggest that NPC^PSA-NCAM+ transplantation intensely suppressed reactive astrocytes activation, thereby promoting a favorable environment for tissue repair (Gonzalez, F. F., McQuillen, P., Mu, D., et al. (2007). Developmental Neuroscience, 29, 321-330).

Ischemic stroke induces adverse microglial response accompanied by tissue damage. Thus, the present inventors examined the microglial activation in ischemic brain tissues at day 26 following transplantation using an ED1 antibody that recognizes CD68 (active microglial marker) (FIG. 4e). Of importance, the number of ED1-positive cells significantly decreased in NPC^PSA-NCAM+ group (P<0.001), and to a lesser extent in MSC-transplanted group (P<0.05) (FIG. 4f). Although hNu+ cells in the striatum were detected after 6 months, there was no sign of teratoma in any of the grafts or other regions of the brains transplanted with NPC^PSA-NCAM+.

***Transplantation of NPC^PSA-NCAM+ Enhanced Angiogenesis in Host Brain***

Endogenous angiogenesis was examined in ischemic brain tissues using $\alpha$-SMA antibody, a smooth muscle actin marker. As a result, it was observed that the number of $\alpha$-SMA-reactive vessels in NPC^PSA-NCAM+-transplanted rats increased around the graft site, compared with those of MSC- and PBS-treated groups (FIG. 5a, b). Quantitative analysis of micro-vessels clearly demonstrated that $\alpha$-SMA+ vessels in NPC^PSA-NCAM+-transplanted rats increased in the infract area, compared with those of MSC- and PBS-treated groups (FIG. 5c) .

When the expression levels of pro-angiogenic marker, angiopoietin-1, in the brain tissues were investigated using RT-PCR at day 7 and 26 after transplantation, the levels were substantially higher in NPC^PSA-NCAM+-transplanted rats at day 26 than other groups (P<0.05) (FIG. 5d), suggesting the induction of angiogenesis by long-term survived NPC^PSA-NCAM+. NPC^PSA-NCAM+-transplanted rats at day 26 also demonstrated an increasing pattern of angiopoietin-1 compared with its level at day 7, whereas angiopoietin-1 in MSCs-treated rats at day 26 did not change or slightly decreased compared with the level at day 7. However, MSCs-treated rats showed an increasing pattern at day 7 compared with that of PBS-treated group (P<0.05).

**Example 2: Treatment Effect of Secretome of Neural Precursor Cells on Ischemic Disease and Neuroinflammaotry Disease**

**Materials and Methods**

*Human ESC-derived NPC<sup>PSA-NCAM+</sup> Cells*

The use of human cells was approved by the Institutional Review Board (IRB No. 4-2008-0643). For neural induction, embryoid bodies (EBs) derived from hESCs and iPSC were cultured for 4 days in suspension with 5 $\mu$M dorsomorphin (DM) (Sigma, St. Louis, MO) and 5-10 $\mu$M SB431542 (SB) (Calbiochem, San Diego, CA) in hESC medium deprived of bFGF (Invitrogen), and then attached on Matrigel-coated dishes (BD Biosciences, Bedford, MA) in 1×N2 (Invitrogen) media supplemented with 20 ng/ml bFGF for the additional 5 days (Kim, D. S., Lee, D. R., Kim, H. S., et al. (2012). Highly pure and expandable PSA-NCAM-positive neural precursors from human ESC and iPSC-derived neural rosettes. PLoSOne, 7, e39715). Neural rosettes that appeared in the center of attached EB colonies were carefully isolated using pulled glass pipettes from the surrounding flat cells. Small rosette clumps were then seeded on Matrigel-coated dishes and cultured in DMEM/F12 supplemented with 1× N2, 1× B27 (Invitrogen) (Kim, D. S., Lee, J. S., Leem, J. W., et al. (2010). Robust enhancement of neural differentiation from human ES and iPS cells regardless of their innate difference in differentiation propensity. Stem Cell Reviews and Reports, 6, 270-281).

Expanded neural rosette cells at 80-90 % confluence were exposed to 10 $\mu$M Y27632 (Sigma) for 1 h to prevent cell death prior to being subjected to MACS procedure. After dissociation using Accutase (Invitrogen), the cells ($1 \times 10^8$ cells or less) were briefly blocked in PBS with 1 % BSA, and then incubated with anti-PSA-NCAM antibody conjugated with microbeads (Miltenyi Biotec) for 15 min at 4□. After extensive washing, the cell suspension was subjected to the MACS procedure, and positively-labeled cells that remained in the column were eluted to a tube with culture media. Isolated NPC<sup>PSA-NCAM+</sup> were re-plated on the culture dish at a density of 4 to $5 \times 10^5$ cells/cm$^2$ in N2B27 medium or NBG medium plus 20 ng/ml of bFGF (1× N2, 0.5× B27, and 0.5× G21 supplement (Gemini BioProducts, West Sacramento, CA)). Culture medium was changed every day and the cells were passaged every 2-3 days.

*Isolation of secretome in human pluripotent stem cell-derived neural precursor cells (NPSs)*

The obtained human pluripotent stem cell-neural precursor cells (PSA-NCAM-positive neural precursor cells) were repeatedly cultured and amplified for 4 passages or more in a Matrigel-coated 60-mm dish with serum removal supplements of N2 (100X-final concentration 1X), B-27 (50X-final concentration 0.5X) and Gem21 (50X-final concentration 0.5X) and bFGF (20 ng/m$\ell$) in a base culture (DMEM/F-12), and then grown in 8-10 dishes until the cells reached 90% confluence. Following removal of the culture liquid, the cells were washed three times with phosphate buffered saline, and cultured for 24 h in a serum-free base culture liquid (DMEM/F12) supplemented with only ITS (100X-final concentration 1X) and bFGF (20 ng/m$\ell$). For a control, the same amount of base culture liquid with the same composition (base culture liquid supplemented with the same amount of ITS and bFGF) was put in a dish without cells, and cultured in an incubator for 24 h, and then collected. The culture liquid was all collected and centrifuged (800 g at 30 min) to remove cellular debris, and then directly frozen in a freezer at -70□, and, when necessary, was thawed before use.

*Establishment of Stroke Model*

In order to measure the neural protective effects against neural damage due to local ischemic stroke, an applied intraluminal suture method was used. This method is a local ischemic stroke model developed by Zia Longa (Zea Longa, et al, Stroke., 1989, 20, 84-91), and an advantage of clinical similarity, unlike other models. For this reason, this model is suitable to search for ischemia-reperfusion mechanism or the screening of effects of several drugs.

After acclimatization for one week, animals (male Sprague-Dawley rat, body weight 250-300 g) were anesthetized using a respiration anesthetic machine, and isoflurane was used for an anesthetic drug. White rats were first subjected to general anesthesia using a mixture gas of 80% $N_2O$ and 20% $O_2$, and 5% isoflurane, which was then maintained at 2-2.5% for anesthesia. For the establishment of stroke model, the left neck skin of the white rats was incised, and then the common carotid artery, external carotid artery, and internal carotid artery were isolated, and the respective arteries were slightly tied with a black silk thread to block the blood flow. The common carotid artery was cut in half, and the 25-mm 4-0 nylon probe with an end of 0.40 mm, obtained by rounding the end of the nylon suture using cautery, was inserted through the cut section. The nylon probe inserted into the external carotid artery was inserted and fixed into the middle cerebral artery via the internal carotid artery. After the probe is inserted at about 18-20 mm from the branch site of the common carotid artery, the origin of the middle cerebral artery was blocked, and then fixed by a thread to permanently occlude the middle cerebral artery. Thereafter, the skin incision site was again sutured, and then the rats were naturally recovered from the anesthesia.

***Injection of Secretome into Stroke Model through Cerebral Artery and Behavioral Test***

After the baseline for the behavioral test was established one day after stroke induction, an insulin syringe needle is inserted into the internal carotid artery via the right external carotid artery in the same manner as stroke model establishment, and through the needle, 0.2 mg/kg (volume 50 $\mu\ell$) of the secretome was intraarterially injected, and the same volume of the culture liquid or phosphate buffered saline (PBS) was administered as a control. After the injection of the secretome liquid, the state of the animals was observed for 14 days. The weight measurement was conducted once before injection and four times after injection, and the behavioral analysis was conducted.

1) Torso twisting test: In order to test the upper body posture considered as the sense of the cerebral cortex and striatum of animals, asymmetric behavior was measured.

2) Beam balance test: The gross vestibulomotor function was evaluated through steady posture of the animals on the narrow beam.

3) Foot-fault test: This test is used when the adjustment (cooperation) and unification (integration) of the motor movement are tested. The foot fault is defined that the paw falls between the grid bars or the rat misplaces a forelimb or hind-limb. The foot fault is symmetric in normal animals.

4) Prehensile Traction test: The prehensile portion of the test involves the rat's ability to hang onto the horizontal rope by its forepaws. The prehensile traction test was used to measure the rat's muscle strength. This test was adapted from similar tests described previously. The steel bar (2-cm diameter, 100-cm length) was placed horizontally 70 cm above the sponge rubber pad (7.5 cm thickness). The rat's forepaws were placed on the steel bar and the animal was released. The animals were allowed to hang onto the steel bar for up to 5 s. Time of falling was noted as well as whether or not the animals brought the rear limb up to the bar with the following scores; score 0-the rat hangs on for 5 s and brings rear limb up, score 1- rat hangs on for 5 s and no rear limb is brought up, score 2- rat hangs on for 3-4 s, score 3-the rat hangs on for 0-2 s.

5) Open-field test: This test is used to find out general walking activity levels. The activity, emotion, and behavioral patterns of the animals were measured by directly measuring the behavioral aspects and characteristics.

6) Modified Neural Severity Score (mNSS): The score is a total score of motor, sensory, balance, reaction, and emotion test values obtained through the above various tests, and the score is calculated by the following criteria (mNSS is measured by adding up scores for each subject).

- Open field test (measuring emotion, activity, and behavioral patterns of animals).

  No movement: 3
  1-20: 2
  21-30: 1
  30 or more: 0

- Prehensile traction test (muscular measurement) 0-5 s: 3

  6-10 s: 2
  11-20 s: 1
  21 s or over: 0

- Beam Balance test (measurement of sense of balance)

  Score 0: 1 = Stable posture
  2 = grasps side of beam and has shaky movement
  Score 1: 3 = one or more paws slip off beam.
  4 = attempts to balance on the beam but falls off
  Score 2: 5 = drapes over the beam but falls off.
  6 = falls off the beam with no attempt to balance

- Foot fault test (Motor cooperation ability)

0-5 s: 0
6-10 s: 1
11-20 s: 2
21 s or above: 3

- Upper body posture test (Asymmetric behavior test)

0: 2
1-4: 1
5 or more: 0

The white rats were anesthetized with zoletil 14 days after ischemic induction, and subjected to lung open and right auricle incision. A needle was injected into the left ventricle, and then, the heart was perfused with PBS using a pump to remove blood flow, and then the brain tissue was extracted. The tissue sections for sample construction were embedded in paraffin on the basis of bregma. For verification of damaged brain tissue, the brain tissue sections were stained with haematoxylin and dehydrated, and the slide was photographed using a digital camera, and then transferred to a computer. The percentage of infarction area was calculated by equation 1 using an image analysis program (image J).

## Equation 1

Percentage of infarct (%) = (the area of the contralateral hemisphere – the intact area of the ipsilateral hemisphere)/ the area of the contralateral hemisphere X 100

### Secretomics

The secretome of neural precursor cells derived from hESC and the secretome of neural precursor cells derived from human iPSC were separated on SDS-PAGE using 4-12% gradient Novex Bis-Tris gel (Invitrogen), and then gels were stained with Gel Code Blue staining reagent (Piece) to show protein bands. The stained gels were cut into 10 bands with the same size, which were then subjected to In-Gel Tryptic Digestion by a known method.

The peptides prepared by the In-gel tryptic digestion were analyzed by the LinearTrap Quadrupole (LTQ) mass spectrometer (Thermo Finnigan) coupled with Nano Ultra Performance liquid chromatography (Eksigent Technologies). Specifically, trypsinized peptides were applied to an analytical column ($75\mu m \times 11cm$) packed with C18 regular $5\mu m$-sized resin. A linear 45min gradient was achieved from 97% solvent A (0.1% formic acid in distilled water) to 60% solvent B (0.1% formic acid in acetonitrile) at a flow rate of $0.3\mu l/min$. The separated peptide ions were electrosprayed into the nano-electrospray ionisation (ESI) source. All MS/MS spectra were acquired by data-dependent scans in which the five most abundant spectra from the full MS scan were selected for fragmentation. The repeat count for dynamic exclusion was set to 1, the repeat duration was 30s, the dynamic exclusion duration was set to 180s, the exclusion mass width was 1.5 Da, and the list of dynamic exclusion was 50.

The identification of peptides and proteins was researched from ipi.HUMAN v3.76 database (89 378 entries) using Turbo-SEQUEST algorithm (Thermo Finnigan). Following database research, the identified peptides and proteins were confirmed using scaffold 2 (Proteome Software). Among the peptides obtained from the SEQUEST search, a set of peptides with a PeptideProphet probability greater than 0.95 was selected. Furthermore, a list of proteins that had a ProteinProphet probability greater than 0.99 and also had more than two unique peptides obtained.

### Statistical Analysis

The statistical significance among groups was obtained using one-way analysis of variance (ANOVA) with Tukey's correction, and a p value < 0.05 was determined to be statistically significant.

### Results

To investigate the alleviation of the disease by the secretome of neural precursor cells in a stoke model, the following three groups were tested for 2 weeks. At 24 h after stroke induction, white rats with the induced disease confirmed by behavior test were arbitrarily allocated into three groups, and an equal volume (50 $\mu l$) of the secretome

(0.2 mg/kg, volume 50 $\mu\ell$), medium, or PBS was injected into the right external carotid artery. The condition and weight of animals were monitored at 3, 7, 10, and 14 after the injection of each material, and behavior analysis was performed.

[Table 1]

| Group | Description |
|---|---|
| PBS control | PBS-treated group after stroke induction |
| Medium control | Stroke animal treated with basal unconditioned Medium |
| Secretome-treated group | Stroke animal treated with conditioned medium (secretome) derived from neural precursor cell culture |

### Ischemic lesion analysis results

The induction of stroke through permanent MCAO in white rats induced extensive brain lesion. The brain was extracted 14 days after the stroke induction, and then the damage and the damaged sites of the brain were confirmed by TTC (2,3,5-triphenyltetrazolium chloride) staining. TTC staining occurs by a reaction with normal mitochondrial oxidative enzyme system in cells, and the damaged mitochondria by ischemic damage are not stained due to the disturbance of the oxidative system, showing white, which can differentiate the damaged sites of the brain.

As shown in FIG. 7, the damage induced by middle cerebral artery occlusion mainly appeared on the cortex and striatum (FIG. 7). The injection of the secretome of neural precursor cells reduced the ischemic lesion area (infarct size). The PBS control showed approximately the damage of 60% of the right brain and the medium control showed about 46%, but the secretome-treated group showed a damaged site of about 29%, which was significantly reduced compared with the controls.

### Body weight analysis results

The stroke induction reduces the motor performance of white rats, the immediate weight loss was advanced by 7 days, and various treatment agents induced the body weight increase through the recovery of motor performance. The injection of the secretome also induced the body weight increase similar to cell transplantation (FIG. 8). The recovery of body weight was notable in recovery of the neural damage. The secretome-treated group showed a significant improvement compared with the PBS control.

### Behavior analysis results

The secretome-treated group showed a statistically significant behavior improvement effect compared with the two controls in the beam balance test, and there was an immediate effect, for example, the effect was exhibited from day 3 post-treatment (FIG. 9).

In addition, the secretome-treated group showed a statistically significant effect compared with the two controls on day 7 post-treatment (injection) in the prehensile traction test.

Further, the injection of the secretome showed the reduction effect in the foot fault frequency in a net (FIG. 9c). The injection of the secretome also showed an improvement in the line cross for measuring the activeness of behavior per unit time

### Modified Neurological Severity Score (mNSS) analysis results

The modified neurological severity score (mNSS) test is a composite table for measuring neurological functions. Motor (muscle state) and sensory (vision, touch, and proprioceptive) items were evaluated. Normal score is 0, and the higher score, the more severe is the dysfunctions. As shown in FIG. 10, the secretome-treated group showed a significantly higher treatment effect (behavior improvement) from the early treatment in the mNSS analysis, compared with the PBS control and the medium control (FIG. 10).

In the mNSS test, the PBS control showed that the average score was 5.4 n day 1 and 5.5 on day 14 after the ischemic induction, indicating that the neurobehavioral disorders caused by stroke was maintained. The medium control showed a temporary behavior improvement effect on day 3 of treatment, but did not exert an additional improvement effect. Whereas, the secretome-treated group showed a continuous behavior improvement effect from day 3 of treatment (mNSS score: 4.5) to day 10 of treatment (mNSS score: 3).

*Secretome analysis results*

The secretome obtained from neural precursor cells derived from iPSCs includes the following proteins: Agrin, annexin A5, BSG(Basigin), biglycan, calponin-3, coactosin-like protein, cofilin-1, collagen alpha-2, cullin-3, destrin, dystroglycan, ephrin-B2, exportin-2, ezrin, fibronectin, fibulin-1, frizzled-related protein, gelatin-3 binding protein, granulins, growh/differentiation factor 11, haptoglobin, hemopexin, high mobility group protein B2, hornerin, importin-9, insulin-like grwoth factor-binding protein 2, Lupus La protein, macrophage migration inhibitory factor, midkine, moesin, neuropilin 2, pleiotrophin, profilin-1, protein DJ-1, radixin, secreted frizzled-related protein-2, septin-11, talin-1, testican, thymopoietin, transgelin-3 and vimentin.

The secretome obtained from neural precursor cells derived from human embryonic stem cells includes the following proteins: Agrin, annexin A2, attractin, biglycan, ceruloplasmin, cofilin-1, collagen alpha-1, coronin-1X, dermicidin, DERP12, eprin-B3, exostosin-2, ezrin, gelatin-3 binding protein, granulins, growh/differentiation factor 11, haptoglobin, hemopexin, high mobility group protein B2, hornerin, insulin-like grwoth factor-binding protein 2, Lupus La protein, midkine, moesin, multiple epidermal growth factor-like domains protein 8, nidogen-1, parathymosin, profilin-2, protein DJ-1, secreted frizzled-related protein-2, secretogranin, talin-1, thymosin beta-4, TGFBI(Transforming grwowth factor-beta-induced protein ig-h3), transgelin and vimentin.

## Claims

1. A composition for use in the treatment of ischemic disease, the composition containing poly-sialylated neural cell adhesion molecule (PSA-NCAM)-positive neural precursor cells as an active ingredient
wherein the PSA-NCAM-positive neural precursor cells are separated from neural rosettes differentiated from pluripotent stem cells
wherein the pluripotent stem cells are embryonic stem cells, or induced pluripotent stem cells (iPSCs),
wherein the ischemic disease is selected from the group consisting of ischemic cerebrovascular disease, ischemic heart disease, myocardial infarction, angina pectoris, lower limb artery ischemic disease, and distal limb ischemic disease.

2. The composition for use according to claim 1, wherein the ischemic disease is ischemic cerebrovascular disease, ischemic heart disease, or myocardial infarction.

3. The composition for use according to claim 1 or 2, wherein the ischemic cerebrovascular disease is ischemic stroke.

## Patentansprüche

1. Zusammensetzung zur Verwendung bei der Behandlung von ischämischer Krankheit,
die Zusammensetzung umfassend polysialyliertes neurales Zellenadhäsionsmolekül (poly-sialylated neural cell adhesion molecule - PSA-NCAM)-positive neurale Vorläuferzellen als einen Wirkstoff,
wobei die PSA-NCAM-positiven neuralen Vorläuferzellen von neuralen Rosetten, die sich von pluripotenten Stammzellen unterscheiden, getrennt sind,
wobei die pluripotenten Stammzellen embryonische Stammzellen oder induzierte pluripotente Stammzellen (induced pluripotent stem cells - iPSC) sind,
wobei die ischämische Krankheit aus der Gruppe bestehend aus ischämischer cerebrovaskulärer Krankheit, ischämischer Herzkrankheit, Myokardinfarkt, Angina pectoris, ischämischer Krankheit der Arterie der unteren Extremitäten und ischämischer Krankheit der distalen Extremitäten ausgewählt ist.

2. Zusammensetzung zur Verwendung nach Anspruch 1, wobei die ischämische Krankheit ischämische cerebrovaskuläre Krankheit, ischämische Herzkrankheit oder Myokardinfarkt ist.

3. Zusammensetzung zur Verwendung nach Anspruch 1 oder 2, wobei die ischämische cerebrovaskuläre Krankheit ischämischer Schlaganfall ist.

## Revendications

1. Composition destinée à être utilisée dans le traitement d'une maladie ischémique, la composition contenant des cellules précurseurs neurales positives pour la molécule d'adhérence cellulaire neurale poly-sialylée (PSA-NCAM) en tant que principe actif,
lesdites cellules précurseurs neurales positives pour la PSA-NCAM étant distinctes des rosettes neurales différenciées à partir de cellules souches pluripotentes,
lesdites cellules souches pluripotentes étant des cellules souches embryonnaires ou des cellules souches pluripotentes induites (iPSC),
ladite maladie ischémique étant choisie dans le groupe constitué par une maladie ischémique cérébrovasculaire, une maladie ischémique cardiaque, un infarctus du myocarde, une angine de poitrine, une maladie ischémique des artères des membres inférieurs et une maladie ischémique des membres distaux.

2. Composition destinée à être utilisée selon la revendication 1, ladite maladie ischémique étant une maladie ischémique cérébrovasculaire, une maladie ischémique cardiaque ou un infarctus du myocarde.

3. Composition destinée à être utilisée selon la revendication 1 ou 2, ladite maladie ischémique cérébrovasculaire étant un accident vasculaire cérébral ischémique.

Fig. 1a

Body weight measurement
and Behavioral evaluation

Cyclosporin A (10mg/kg/24 hr)

-3  -2  -1  0       3       7       13      17      24  26

pMCAo  Transplantation      Sacrifice              Sacrifice

## Fig. 1b

## Fig. 1c

PBS          NPC<sup>PSA-NCAM+</sup>          MSC

Fig. 2a

## Fig. 2b

Fig. 2c

Fig. 2d

## Fig. 2e

Fig. 2f

Fig. 3b

Fig. 3c

## Fig. 3d

Fig. 4a

## Fig. 4b

Fig. 4c

## Fig. 4d

## Fig. 4e

# Fig. 4f

## Fig. 5a

## Fig. 5b

Fig. 5c

## Fig. 5d

Fig. 6

# Fig. 7

EP 3 162 372 B1

## Fig. 8

## Fig. 9a

## Fig. 9b

## Fig. 9c

## Fig. 9d

## Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20120136638
- WO 2008070513 A1
- US 481656 A
- KR 15812792
- KR 1020140080009

### Non-patent literature cited in the description

- **CAPLAN, A. I. ; DENNIS, J. E.** *Journal of Cellular Biochemistry,* 2006, vol. 98, 1076-1084
- **CHEN, J. ; LI, Y. ; KATAKOWSKI, M. et al.** *Journal of Neuroscience Research,* 2003, vol. 73, 778-786
- **KOPEN, G. C. ; PROCKOP, D. J. ; PHINNEY, D. G.** *Proceedings of the National Academy of Sciences,* 1999, vol. 96, 10711-10716
- **BAE, K. S. ; PARK, J. B. ; KIM, H. S. ; KIM, D. S. ; PARK, D. J. ; KANG, S. J.** *Yonsei Medical Journal,* 2011, vol. 52, 401-412
- **CHO, S. R. ; KIM, Y. R. ; KANG, H. S. et al.** *Cell Transplantion,* 2009, vol. 18, 1359-1368
- **CAPONE, C. ; FRIGERIO, S. ; FUMAGALLI, S. et al.** *PLoSOne,* 2007, vol. 7, e373
- **KIM, DAE - SUNG et al.** Highly Pure and Expandable PSA-NCAM-Positive Neural. *PLOS ONE,* July 2012, vol. 7 (7
- **DIBAJNIA et al.** Role of neural precursor cells in promoting repair following stroke. *ACTA PHARMACOLOGICA SINICA,* 2013, vol. 34 (1), 78-90
- Increased generation of neuronal progenitors after ischemic injury in the aged adult human forebrain. **MACAS JADRANKA et al.** JOURNAL OF NEUROSCIENCE. SOCIETY FOR NEUROSCIENCE, 30 November 2006, vol. 26, 13114-13119
- The modulations of NCAM polysialylation state that follow transient global ischemia are brief on neurons but enduring on glia. **FOX GERARD B et al.** JOURNAL OF NEUROPATHOLOGY AND EXPERIMENTAL NEUROLOGY. LIPPINCOT WILLIAMS AND WILKINS, 31 January 2001, vol. 60, 132-140
- Three steps of neural stem cells development in gerbil dentate gyrus after transient ischemia. **IWAI MASANORI et al.** JOURNAL OF CEREBRAL BLOOD FLOW & METABOLISM. NATURE PUBLISHING GROUP, 31 March 2002, vol. 22, 411-419
- DATABASE WPI. 2012-R77360
- **TROPEPE V et al.** *Neuron,* 2001, vol. 30, 6578
- **YING QL et al.** *Nat Biotechnol.,* 2003, vol. 21, 183186
- **KOHLER ; MILSTEIN.** *Journal of Immunology,* 1976, vol. 6, 511-519
- **CLACKSON et al.** *Nature,* 1991, vol. 352, 624-628
- **MARKS et al.** *J. Mol. Biol.,* 1991, vol. 222 (58), 1-597
- **HARLOW, E. ; LANE, D.** Using Antibodies: A Laboratory Manual. Cold Spring Harbor Press, 1999
- **ZOLA, H.** Monoclonal Antibodies: A Manual of Techniques. CRC Press, Inc, 1984
- **COLIGAN.** CURRENT PROTOCOLS IN IMMUNOLOGY. Wiley/Greene, 1991
- Remington's Pharmaceutical Sciences. 1995
- **KIM, D. S. ; LEE, J. S. ; LEEM, J. W. et al.** *Stem Cell Reviews and Reports,* 2010, vol. 6, 270-281
- **GONZALEZ, F. F. ; MCQUILLEN, P. ; MU, D. et al.** *Developmental Neuroscience,* 2007, vol. 29, 321-330
- **KIM, D. S. ; LEE, D. R. ; KIM, H. S. et al.** Highly pure and expandable PSA-NCAM-positive neural precursors from human ESC and iPSC-derived neural rosettes. *PLoSOne,* 2012, vol. 7, e39715
- **KIM, D. S. ; LEE, J. S. ; LEEM, J. W. et al.** Robust enhancement of neural differentiation from human ES and iPS cells regardless of their innate difference in differentiation propensity. *Stem Cell Reviews and Reports,* 2010, vol. 6, 270-281
- **ZEA LONGA et al.** *Stroke,* 1989, vol. 20, 84-91

SEQUENCE LISTING

<110> INDUSTRY-ACADEMIC COOPERATION FOUNDATION, Yonsei University

<120> A Composition for Treating Ischemic Diseases or Neuroinflammatory Diseases Comprising Neural Precursor Cells or Therir Secretome

<130> PP150076

<140> 15812792.8
<141> 2015-06-26

<150> KR 10-2014-0080009
<151> 2014-06-27

<160> 2

<170> KopatentIn 2.0

<210> 1
<211> 22
<212> DNA
<213> Artificial Sequence

<220>
<223> forward primer for Angiopoietin-1

<400> 1
tgtgtccatc agctccagtt gc        22

<210> 2
<211> 22
<212> DNA
<213> Artificial Sequence

<220>
<223> reverse primer for Angiopoietin-1

<400> 2
cggctaccat gctcgagata gg        22